# EUROPEAN PATENT SPECIFICATION

(11) **EP 3 794 107 B1**
(45) Date of publication and mention of the grant of the patent: **29.04.2026**
(21) Application number: 19724199.5
(22) Date of filing: 16.05.2019
(51) Int. Cl.: C12N 5/071

(54) **METHOD OF CULTURING PROLIFERATIVE HEPATOCYTES**
VERFAHREN ZUR KULTIVIERUNG VON PROLIFERATIVEN HEPATOZYTEN
PROCÉDÉ DE CULTURE D'HÉPATOCYTES PROLIFÉRANTS

(30) Priority: 16.05.2018 EP 18305603
(43) Date of publication of application: 24.03.2021
(73) Proprietor: Institut National de la Santé et de la Recherche Médicale (INSERM), 75654 Paris Cedex 13 (FR); Université de Rennes, 35042 Rennes (FR); Ecole des Hautes Etudes en Santé Publique (EHESP), 35043 Rennes cedex (FR)
(72) Inventor: BAFFET, Georges, 35650 Le Rheu (FR); LANGOUET, Sophie, 35700 Rennes (FR); EZAN, Frédéric, 35000 Rennes (FR); ROSE, Sophie, 35000 Rennes (FR); CUVELLIER, Marie, 35000 Rennes (FR)
(74) Representative: Icosa
(86) International application number: PCT/EP2019/062649
(87) International publication number: WO 2019/219828

(56) References cited:
- YUJI NISHIKAWA ET AL: "Hepatocytic Cells Form Bile Duct-like Structures within a Three-Dimensional Collagen Gel Matrix", EXPERIMENTAL CELL RESEARCH, 15 March 1996 (1996-03-15), United States, pages 357 - 371, XP055491836, Retrieved from the Internet <URL:https://ac.els-cdn.com/S0014482796900919/1-s2.0-S0014482796900919-main.pdf?_tid=c7b209e9-9fb6-4b8a-8cf0-76634eb00c75&acdnat=1531299724_07898919ecbe5a6751165fcfb1016da6> DOI: 10.1006/excr.1996.0091
- JÉRÉMY BOMO ET AL: "Increasing 3D Matrix Rigidity Strengthens Proliferation and Spheroid Development of Human Liver Cells in a Constant Growth Factor Environment :", JOURNAL OF CELLULAR BIOCHEMISTRY, vol. 117, no. 3, 8 September 2015 (2015-09-08), pages 708 - 720, XP055490868, ISSN: 0730-2312, DOI: 10.1002/jcb.25356
- RUI M. TOSTÕES ET AL: "Human liver cell spheroids in extended perfusion bioreactor culture for repeated-dose drug testing", HEPATOLOGY, vol. 55, no. 4, 1 April 2012 (2012-04-01), US, pages 1227 - 1236, XP055492253, ISSN: 0270-9139, DOI: 10.1002/hep.24760
- CATHERINE C. BELL ET AL: "Characterization of primary human hepatocyte spheroids as a model system for drug-induced liver injury, liver function and disease", SCIENTIFIC REPORTS, vol. 6, no. 1, 4 May 2016 (2016-05-04), XP055451253, DOI: 10.1038/srep25187
- POPNIKOLOV N K ET AL: "In vivo growth stimulation of collagen gel embedded normal human and mouse primary mammary epithelial cells", JOURNAL OF CELLULAR PHYSIOLOGY, WILEY SUBSCRIPTION SERVICES, INC, US, vol. 163, no. 1, 1 April 1995 (1995-04-01), pages 51 - 60, XP002601765, ISSN: 0021-9541
- MICHELLE B. CHEN ET AL: "A 3D microfluidic platform incorporating methacrylated gelatin hydrogels to study physiological cardiovascular cell-cell interactions", LAB ON A CHIP, vol. 13, no. 13, 1 January 2013 (2013-01-01), pages 2591, XP055310753, ISSN: 1473-0197, DOI: 10.1039/c3lc00051f
- X. CUI ET AL: "Advances in multicellular spheroids formation", JOURNAL OF THE ROYAL SOCIETY. INTERFACE, vol. 14, no. 127, 1 February 2017 (2017-02-01), GB, pages 20160877, XP055599271, ISSN: 1742-5689, DOI: 10.1098/rsif.2016.0877

## Description

### FIELD OF INVENTION

The present invention relates to the field of primary human hepatocyte culture, and in particular 3D primary human hepatocyte culture.

### BACKGROUND OF INVENTION

Hepatocytes are the major parenchymal cells of the liver and represent up to 70-85% of the liver mass. Hepatocytes are highly differentiated cells that carry out most of the hepatic functions, which pertain notably to metabolism, detoxification and systemic homeostasis. In the liver, hepatocytes normally are long-lived quiescent cells. However, upon injury or loss of functional mass, hepatocytes are able to proliferate, thus allowing liver regeneration.

The establishment of *in vitro* cultures of hepatocytes has long been pursued with the aim of developing *in vitro* models able to faithfully recapitulate key liver functions. Such models can be used in research to understand and study normal liver functions. They can also be used to understand and study liver pathologies, for example viral hepatitis, and assess the ability of candidate drugs to restore liver functions. Moreover, given the central role of the liver in metabolism and detoxification, *in vitro* cultures of hepatocytes are relevant models to predict the metabolism and toxicity of new drugs. In particular, cultures of primary human hepatocytes (PHH) are considered the gold standard model for assessing *in vitro* drug metabolism, drug-drug interaction and hepatotoxicity. Finally, *in vitro* cultures of primary human hepatocytes (PHH) also represent a promising approach in the treatment of liver diseases or disorders, for example through the development of extracorporeal devices, hepatocyte transplantation or transplantation of three-dimensional (3D) hepatocyte structures as an alternative to organ transplantation.

Thus, numerous hepatocyte culture systems have been designed, starting with the conventional two-dimensional (2D) monolayer cultures of primary hepatocytes, in particular of primary human hepatocytes. However, in such cultures, the primary hepatocytes de-differentiate and rapidly lose hepatocyte-specific functions. Moreover, conventional 2D monolayer cultures do not allow the long-term survival of primary hepatocytes. More complex culture systems have been developed, notably to allow longer survival and better maintenance of hepatocyte-specific functions. For example, primary hepatocytes have been cultured in a sandwich configuration, between two layers of gelled extracellular matrix proteins. Hepatocytes have also been co-cultured, for instance with non-parenchymal liver cells (Baffet *et al.,* 1991), hepatocellular carcinoma (HCC) cells (Jang *et al.,* 2016) or 3T3 fibroblasts (Berger *et al.,* 2016). More recently, three-dimensional (3D) cultures have been showed to be particularly suited for obtaining hepatocytes with a stable phenotype, able to retain morphology, viability and hepatocyte-specific functions. In particular, primary human hepatocyte free sphere-shaped aggregates can be obtained, for example when hepatocytes are cultured in ultra-low attachment plates (Bell *et al.,* 2016) or when hepatocytes are encapsulated or embedded in a matrix or a scaffold, such as a polysaccharide scaffold, for example alginate (WO2013/087843). Critically, none of the above-mentioned culture systems enable proliferation of the cultured primary human hepatocytes.

Cultures of primary hepatocytes are also described in Nishikawa *et al.,* 1996; Bomo *et al.,* 2016; and Tostões *et al.,* 2012. Nishikawa *et al.,* 1996 disclose culturing primary rat or mouse hepatocytes or immortal liver epithelial cell lines embedded in a collagen matrix with the aim to obtain bile duct-like structures from rat or mouse hepatocytes (Exp Cell Res. 1996 Mar 15;223(2):357-71). Bomo *et al.,* 2016 describe culturing primary hepatocytes, in particular primary human hepatocytes, embedded in a collagen matrix (J Cell Biochem. 2016 Mar;117(3):708-20). Tostões *et al.,* 2012 disclose conducting primary cultures of freshly isolated human hepatocytes in an automated perfusion bioreactor, in order to obtain and maintain human hepatocyte spheroids (Hepatology. 2012 Apr;55(4):1227-36). None of these documents provide any information regarding the proliferation of cultured primary human hepatocytes.

Proliferation can be observed using alternative cell systems, such as hepatocyte-like cell lines, either originating from tumors or obtained by oncogenic immortalization (*i.e.,* by cell transformation), or stem-cell derived hepatocyte-like cells. For example, HuH-7 and HepG2 are hepatocyte-like cell lines derived from human hepatocellular carcinoma with little differentiated functions. Fa2N-4 and Hepa RG are human immortalized cell lines, the latter retaining many characteristics of primary human hepatocytes. However, even with these alternative cell systems, proliferation and differentiation are usually mutually exclusive. Moreover, cancer cell lines and immortalized cell lines do not constitute the optimal *in vitro* model to recapitulate physiological human liver biology.

To date, proliferation of cultured primary hepatocytes has only been observed with rodent hepatocytes. Spontaneous proliferation has been observed with mouse hepatocytes (Frémin *et al.,* 2007) while stimulation with a growth factor has been able to induce proliferation of rat hepatocytes. For example, US 2005/0244959 describes a method to induce active *in vitro* proliferation of rat hepatocytes through the addition of at least a cytokine and a growth factor, preferably TNFα and EGF, to the rat hepatocyte culture. However, as with cancer cell lines and immortalized cell lines, rodent hepatocyte cultures do not constitute the optimal *in vitro* model to recapitulate physiological human liver biology.

Thus, studying and taking advantage of the proliferative capacity of hepatocytes for pharmaceutical or therapeutic applications through the use of an *in vitro* model remain a major challenge. In particular, there is still a need for a 3D culturing method enabling the *in vitro* proliferation of human primary hepatocytes.

The present invention thus relates to a method of culturing primary human hepatocytes (PHH) allowing to obtain spheroids comprising proliferative PHH, said proliferative PHH retaining their phenotype, *e.g.,* their differentiated state, and their functions. The method of the invention comprises a first step of culturing the PHH in a non-adherent culture vessel, preferably a low or ultra-low attachment culture vessel, a second step of embedding the PHH in a collagen matrix or in a gelatin matrix, in particular a GelMa matrix, and a third step of culturing the PHH embedded in the collagen matrix or in the gelatin matrix, in particular in the GelMa matrix. The method of the invention allows to obtain proliferative PHH spheroids, *i.e.,* acinus-like structures with a hollow lumen, comprising proliferative primary human hepatocytes. The present invention also relates to a spheroid comprising proliferative primary human hepatocytes and uses thereof, for example for engineering an artificial liver model or an artificial liver organ, or for assessing *in vitro* the toxicity and/or the effects of a drug or a compound.

### SUMMARY

The present invention relates to a method of culturing primary human hepatocytes (PHH) to obtain spheroids comprising proliferative PHH, said method comprising:
a) first culturing the PHH in a non-adherent culture vessel;
b) then transferring the PHH to a culture medium comprising collagen at a concentration ranging from about 0.75 mg/mL to about 3 mg/mL or gelatin at a concentration ranging from about 15% (w/v) to about 40% (w/v) or methacrylated gelatin (GelMa) at a concentration ranging from about 1% (w/v) to about 10% (w/v), thereby embedding the PHH in a collagen matrix or in a gelatin matrix; and
c) culturing the PHH embedded in the collagen matrix or in the gelatin matrix;
thereby obtaining spheroids comprising proliferative PHH, wherein said spheroids have an acinus-like structure with a hollow lumen.

In one embodiment, at step b) of the method as described hereinabove, the PHH are transferred to a culture medium comprising fibrillar collagen selected from the group consisting of type I collagen, type II collagen, type III collagen, type V collagen, type VI collagen, type XI collagen, type XXIV collagen, type XXVII collagen and any mixtures thereof. In one embodiment, the fibrillar collagen is selected from the group consisting of type I collagen, type III collagen, and any mixtures thereof.

In one embodiment, at step b) of the method as described hereinabove, the PHH are transferred to a culture medium comprising methacrylated gelatin (GelMa) at a concentration of about 5% (w/v).

In one embodiment, at step a) of the method as described hereinabove, the PHH are cultured in a non-adherent culture vessel for a period ranging from about 1h to about 96h.

In one embodiment, at step a) the non-adherent culture vessel is a low or ultra-low attachment culture vessel characterized by the presence of a coating that prevents cell attachment to the inner surface of the culture vessel.

In one embodiment, at step c) of the method as described hereinabove, the PHH embedded in the collagen or gelatin matrix are cultured for at least about 2 days.

In one embodiment, the method as described hereinabove further comprises:
d) adding an inhibitor of the MAPK MEK1/2-ERK1/2 pathway to the culture of PHH embedded in the collagen or gelatin matrix, thereby inducing one or more additional wave(s) of proliferation of the PHH.

In one embodiment, the method as described hereinabove further comprises:
e) incubating the spheroids comprising proliferative PHH embedded in the collagen or gelatin matrix with a collagenase or an enzyme mixture with collagenolytic activity, thereby isolating the spheroids comprising proliferative PHH from the collagen or gelatin matrix.

The present invention also relates to a spheroid comprising proliferative primary human hepatocytes (PHH) embedded in a collagen or gelatin matrix susceptible to be obtained with the method as described hereinabove, wherein said spheroid has an acinus-like structure with a hollow lumen. In one embodiment, said spheroid is embedded in a methacrylated gelatin (GelMa) matrix.

In one embodiment, the spheroid has a diameter ranging from about 20 µm to about 130 µm. In one embodiment, the spheroid comprises from about 5 to about 100 proliferative PHH.

The present invention also relates to the use of the spheroid as described hereinabove for engineering an artificial liver model or an artificial liver organ.

The present invention also relates to the use of the spheroid as described hereinabove for assessing *in vitro* the liver toxicity, in particular liver genotoxicity, and/or the effects of a drug or a compound. In one embodiment, said use is for assessing *in vitro* the liver genotoxicity of a drug or a compound.

The present invention also relates to an *in vitro* method of assessing the toxicity and/or the effects of a drug or a compound, the method comprising:
a) obtaining spheroids comprising proliferative primary human hepatocytes (PHH) according to the method as described hereinabove, wherein said spheroids have an acinus-like structure with a hollow lumen;
b) contacting the spheroids comprising proliferative PHH with a drug or a compound; and
c) assessing the toxicity, genotoxicity and/or the effects of the drug or the compound on the spheroids comprising proliferative PHH.

### DEFINITIONS

In the present invention, the following terms have the following meanings:
- "**About**" preceding a figure encompasses plus or minus 10%, or less, of the value of said figure. It is to be understood that the value to which the term "about" refers is itself also specifically, and preferably, disclosed.
- "**Animal cells**" refer to cells originating from a metazoan. The animal cells may be cells originating from a vertebrate, which may be a fish, an amphibian, a reptile, a bird, or a mammal. The animal cells may be mammalian cells, preferably human cells. According to the present invention, the animal cells to be cultured with the method of the invention are primary human hepatocytes (PHH).
- "**Collagen**" refers to a heterogeneous superfamily of extracellular matrix glycoproteins generally involved in the formation of supramolecular networks. Collagens are characterized by the presence of three α chains, that may or may not be identical. The three α chains form a triple-helical domain, with the triple-helical structure corresponding to a right-handed superhelix resulting from the intertwining of the collagenous domains of said three α chains, each of which adopts a polyproline II-like left-handed conformation. As used herein, the term "collagen" encompasses the different sub-families of collagens, including fibrillar collagens; non-fibrillar collagens such as fibril associated collagens and related collagens; beaded filament forming collagens; short-chain collagens and associated proteins; basement membrane collagens and associated collagens; transmembrane collagens and collagen-like proteins; and other collagens and collagen-like proteins. As used herein, the term "collagen" also encompasses truncated collagens. Truncated collagens notably encompass collagens hydrolyzed into shorter peptides and may also be referred to as gelatin.
- **"Collagen matrix"** refers to the three-dimensional matrix resulting from the formation of cross-linked structures, *i.e.,* hydrogel, generated by the polymerization of collagen, in particular of fibrillar collagen. Thus, in one embodiment, a fibrillar collagen matrix is a three-dimensional matrix resulting from the formation of cross-linked structures generated by the polymerization of fibrillar collagen. As used herein, the term "collagen matrix" may also refer to a three-dimensional matrix generated from truncated collagens, *i.e*., gelatin. Alternatively, the three-dimensional matrix generated from truncated collagens, *i.e.,* gelatin, may be referred to as "gelatin matrix" .
- **"Isolated",** as in **"isolated spheroid",** refers to a spheroid that has been separated from its culture environment. Thus, an isolated spheroid may refer to a spheroid that is not embedded in a collagen matrix or in a gelatin matrix.
- **"Gelatin"** refers to a collagen derivative, *i.e*., collagen hydrolyzed into shorter peptides corresponding to truncated collagen. Gelatin can also be defined as denatured collagen. Gelatin is obtained by hydrolysis of collagen such as type I collagen, in particular acidic or alkaline hydrolysis, which unfolds the triple-helical structures of collagen and results in the formation of shorter peptides. Gelatin thus possesses a very similar chemical composition to that of collagen, but a less ordered macromolecular structure. According to a particular embodiment, the gelatin of the invention is methacrylated gelatin (GelMa).
- **"Gelatin matrix"** refers to the three-dimensional matrix resulting from the formation of cross-linked structures, *i.e.,* hydrogel, generated by the polymerization of gelatin, that is to say by the polymerization of truncated collagen. According to a particular embodiment, the gelatin of the invention is methacrylated gelatin (GelMa) and the gelatin matrix of the invention is a GelMa matrix.
- **"GelMa",** "**Gel-MA**", or "**Gel-Ma**" refers to methacrylated gelatin, also known as gelatin methacryloyl, gelatin methacrylate or gelatin methacrylamide. GelMa is obtained by the direct reaction of gelatin with methacrylic anhydride, resulting in modification of lysine and hydroxyl residues with methacrylamide and methacrylate side groups (Yue et al., Biomaterials. 2015 Dec;73:254-71).
- "**GelMa matrix**" refers to the three-dimensional matrix resulting from the formation of crosslinked structures, *i.e.,* hydrogel, generated by the polymerization of the methacrylated gelatin induced by a photoinitiator.
- "**Hydrogel**" refers to a water-swollen network of cross-linked hydrophilic polymers. As used herein, a hydrogel according to the invention mimics salient elements of native extracellular matrices (ECMs) and is thus suitable for use in a method of culturing animal cells.
- "**Primary cells**" refer to cells taken directly from living, non-cancerous tissue, such as for example, cells obtained from a tissue sample of a subject or from a biopsy of a subject.
- "**Proliferation marker**" refers to a marker that is specifically detected in animal cells undergoing cell division, *i.e.,* in proliferative animal cells. Examples of proliferation markers include incorporation of nucleoside analogs such as BrdU (5-bromo-2'-deoxyuridine) or EdU (5-ethynyl-2'-deoxyuridine) in newly synthesized DNA during DNA replication, expression of proteins specifically present in proliferative animal cells such as nuclear proteins (*e.g.,* Ki67, PCNA), cell cycle proteins (e*.g.,* cyclin D1, Cdk2, PCNA, P21, P27). Proliferation may be assessed through the detection of the incorporation of nucleoside analogs such as BrdU or EdU in DNA. As used herein, BrdU or EdU positive animal cells (BrdU⁺ or EdU⁺ animal cells) refer to animal cells in which incorporation of BrdU or EdU was detected. Methods for detecting DNA incorporation of a nucleoside analog in animal cells are well-known to those skilled in the art. Proliferation may be assessed through the detection of the expression of proteins specifically present in proliferative animal cells. As used herein, animal cells positive for a protein are animal cells expressing said protein. For example, animal cells positive for cyclin D1 (cyclin D1+ cells) are animal cells in which expression of cyclin D1, respectively, was detected. Methods for detecting expression of proteins in animal cells are well-known to those skilled in the art and include methods for measuring mRNA levels, for example by RT-PCT or RT-qPCR, and methods for measuring the protein expressions and/or levels, for example by western blotting and immunolocalizations or by immunohistochemistry.
- **"Proliferative primary hepatocytes**" refer to primary hepatocytes able to divide *in vitro,* and thus to proliferate *in vitro.* In one embodiment, primary hepatocytes are proliferative if at least about 10%, 15%, 20%, 25%, 30%, 35%, 40%, 45%, 50%, 55%, 60%, 65%, 70%, 75%, or 80%, preferably at least about 20%, more preferably at least about 30%, even more preferably at least about 40%, of the total number of primary hepatocytes in culture are able to proliferate *in vitro,* for example in the modified William's E medium (WE HH) as defined hereafter at about 37°C and about 5% CO₂, with humidity 80-100%, preferably 85-95%. In one embodiment, proliferation of primary human hepatocytes in culture according to the method of the invention may be assessed through the measurement of BrdU or EdU incorporation and/or through the detection of Ki67 positive (Ki67⁺) primary hepatocytes, *i.e..,* primary hepatocytes expressing Ki67. Alternatively, in one embodiment, proliferation of primary human hepatocytes in culture according to the method of the invention may be assessed through the measurement of the expression of cell cycle markers such as Cdk2, cyclin D1, P21 and/or P27. In one embodiment, the total number of primary human hepatocytes in culture is assessed through the detection of albumin, with cells expressing albumin, *i.e.,* albumin positive cells (Alb⁺), being considered as primary hepatocytes. In a particular embodiment, primary human hepatocytes are proliferative if at least about 10%, 15%, 20%, 25%, 30%, 35%, 40%, 45%, 50% or more, preferably at least about 20%, more preferably at least about 40%, of the total number of Alb⁺ primary hepatocytes in culture are positive for a proliferation marker such as, for example, BrdU incorporation, EdU incorporation, Ki67 expression, or cyclin D1 expression. In one embodiment, proliferative primary human hepatocytes are obtained after at least 1, 2, 3, 4, 5, 6, 7, 8, 9, 10, 11, 12, 13, 14, or 15 days, preferably at least 2 days, of 3D culture in a collagen or gelatin matrix according to the method of the invention. In one embodiment, proliferative primary human hepatocytes are obtained between 2 days and at least 15 days of 3D culture in a collagen or gelatin matrix according to the method of the invention. In one embodiment, proliferative primary human hepatocytes in 3D culture in a collagen matrix according to the method of the invention are able to undergo at least two waves of proliferation, preferably with a first wave between day 2 and day 7 of 3D culture and a second wave between day 8 and day 15 of 3D culture. In one embodiment, one or more additional wave(s) of proliferation are induced by adding an inhibitor of the MAPK MEK1/2-ERK1/2 pathway to the culture of primary human hepatocytes embedded in the collagen or gelatin matrix according to the invention.
- "**Spheroids**" refers to non-adherent, multi-cell 3D structures formed in culture by animal cells which self-assemble. 3D structures formed in culture by animal cells may also be referred to as "organoids". As used herein, the term "spheroids" refers to sphere-shaped aggregates of primary human hepatocytes. Primary human hepatocytes in culture according to the method of the invention form spheroids which have an acinus-like structure with a hollow lumen, and may also be referred to as hepatospheres or as organoids. Thus, the spheroid according to the present invention appears as a sphere delimited by a single layer of well-organized primary human hepatocytes, forming an acinus-like structure with a hollow lumen. In one embodiment, the spheroid according to the present invention has a diameter ranging from about 50 µm to about 150 µm.
- "**3D culture"** refers to the culture of primary human hepatocytes embedded in a collagen matrix or in a gelatin matrix, in particular a GelMa matrix, according to the method of the invention. According to the invention, the primary human hepatocytes are cultured in a collagen or gelatin matrix after having first been incubated in a non-adherent culture vessel, such as a low or ultra-low attachment culture vessel, and then transferred to a culture medium comprising collagen or truncated collagen, *i.e.,* gelatin. Thus, as used herein, "3D culture" corresponds to the third step of the method of the invention.
- "**3D animal cell structures comprising proliferative animal cells**" refer to 3D aggregate of proliferative animal cells, *i.e.,* animal cells able to proliferate *in vitro.* According to the present invention, the 3D animal cell structures are spheroids comprising proliferative PHH, said spheroids having an acinus-like structure with a hollow lumen.

### DETAILED DESCRIPTION

The present invention relates to a method of culturing primary human hepatocytes (PHH) comprising:
a) first culturing the PHH in a non-adherent culture vessel;
b) then transferring the PHH to a culture medium comprising collagen at a concentration ranging from about 0.75 mg/mL to about 3 mg/mL, or truncated collagen, namely gelatin, at a concentration ranging from about 15% (w/v) to about 40% (w/v), or methacrylated gelatin (GelMa) at a concentration ranging from about 1% (w/v) to about 10% (w/v); thereby embedding the PHH in a collagen or gelatin matrix; and
c) culturing the PHH embedded in the collagen or gelatin matrix;
thereby obtaining spheroids comprising proliferative PHH, wherein said spheroids have an acinus-like structure with a hollow lumen.

According to one embodiment, the present invention relates to a method of culturing PHH comprising:
a) first culturing the PHH in a non-adherent culture vessel;
b) then transferring the PHH to a culture medium comprising collagen at a concentration ranging from about 0.75 mg/mL to about 3 mg/mL; thereby embedding the PHH in a collagen matrix; and
c) culturing the PHH embedded in the collagen matrix;
thereby obtaining spheroids comprising proliferative PHH, wherein said spheroids have an acinus-like structure with a hollow lumen.

According to one embodiment, the present invention relates to a method of culturing PHH comprising:
a) first culturing the PHH in a non-adherent culture vessel;
b) then transferring the PHH to a culture medium comprising truncated collagen, namely gelatin, at a concentration ranging from about 15% (w/v) to about 40% (w/v); thereby embedding the PHH in a gelatin matrix; and
c) culturing the PHH embedded in the gelatin matrix;
thereby obtaining spheroids comprising proliferative PHH, wherein said spheroids have an acinus-like structure with a hollow lumen.

In one embodiment, the present invention relates to a method of culturing PHH cells comprising:
a) first culturing the PHH in a non-adherent culture vessel;
b) then transferring the PHH to a culture medium comprising methacrylated gelatin (GelMa) at a concentration ranging from about 1% (w/v) to about 10% (w/v); thereby embedding the PHH in a GelMa matrix; and
c) culturing the PHH embedded in the GelMa matrix;
thereby obtaining spheroids comprising proliferative PHH, wherein said spheroids have an acinus-like structure with a hollow lumen.

According to one embodiment, PHH are proliferative if at least about 10%, 15%, 20%, 25%, 30%, 35%, 40%, 45%, 50%, 55%, 60%, 65%, 70%, 75%, or 80%, preferably at least about 20%, more preferably at least about 40%, of the total number of PHH are positive for a proliferation marker such as, for example, positive for BrdU incorporation (BrdU⁺), positive for EdU incorporation (EdU⁺), positive for Ki67 expression (Ki67⁺), or positive for cyclin D1 expression (cyclin D1⁺).

In one embodiment, the PHH as described hereinabove are not human embryonic stem cells.

According to the present invention, the animal cells to be cultured with the method of the invention are primary human hepatocytes (PHH).

In one embodiment, the PHH to be cultured with the method of the invention are isolated PHH. In one embodiment, the PHH to be cultured with the method of the invention are obtained from a sample of tissue previously taken from a subject, such as, for example, from a biopsy previously taken. In one embodiment, the PHH to be cultured with the method of the invention are kept frozen after having been obtained from a sample of liver tissue previously taken from a subject.

According to the method of the invention, the PHH are first cultured in a non-adherent culture vessel. Without wishing to be bound by any theory, the Applicant suggests that first culturing the PHH in a non-adherent culture vessel enables the PHH to form aggregates and thus enhances cell-cell interactions. Cell-cell interactions are essential for the subsequent development, structuration and growth of spheroids comprising proliferative PHH.

The Applicant indeed showed that directly adding PHH to a culture medium comprising collagen and culturing the PHH thus embedded in a collagen matrix without first culturing the PHH in a non-adherent culture vessel did not allow to obtain spheroids comprising proliferative PHH. Indeed, the Applicant showed that primary human hepatocytes must first be cultured in a non-adherent culture vessel before being transferred to a culture medium comprising collagen or gelatin, in particular methacrylated gelatin (GelMa), and cultured in a collagen matrix or in a gelatin matrix, in particular a GelMa matrix, in order to obtain spheroids comprising proliferative primary human hepatocytes.

As used herein, the term "non-adherent culture vessel" refers to a culture vessel that is not conductive to the attachment of cells, in particular animal cells, to said vessel. In other words, cells, in particular animal cells, being cultured in a non-adherent culture vessel do not attach, or very little, and do not spread to the inner surface of said culture vessel (*i.e.,* to the walls and/or bottom of said culture vessel).

Non-adherent culture vessels include culture vessels made of glass, untreated culture vessels, low attachment culture vessels, and ultra-low attachment culture vessels.

As used herein, the term "untreated culture vessel" refers to a culture vessel that did not undergo the chemical treatment or the coating commonly applied to culture vessels in order to enhance cell attachment to the culture vessels. In the field of cell culture, "treated culture vessel" commonly refers to a culture vessel, such as a plate or a dish, usually made of polystyrene, that underwent a chemical treatment or a coating in order to enhance the attachment of cells, in particular animal cells, to the inner surface of the culture vessel.

Indeed, polystyrene is a very hydrophobic polymer to which cells, in particular animal cells, have difficulty attaching.

In one embodiment, the non-adherent culture vessel as described hereinabove is selected from the group comprising or consisting of culture vessels made of glass, untreated culture vessels, low attachment culture vessels and ultra-low attachment culture vessels; preferably said non-adherent culture vessel is a low or ultra-low attachment culture vessel.

Examples of low and ultra-low attachment culture vessels include low and ultra-low attachment dishes, low and ultra-low attachment flasks and low and ultra-low attachment plates, such as, for example, low and ultra-low attachment multi-well plates or low and ultra-low attachment microplates.

Low attachment culture vessels and ultra-low attachment culture vessels are characterized by the presence of a coating, usually a hydrophilic gel, that is covalently bound to the inner surface of the culture vessel. Said coating inhibits specific and nonspecific immobilization and thus prevents cell attachment to the inner surface of the culture vessel, thereby maintaining cells into a suspended state.

Low and ultra-low attachment culture vessels, in particular low attachment plates (LAP) and ultra-low attachment (ULA) plates, are readily available from suppliers and include, for example, Corning^{®} Costar^{®}, InSphero^{®}, S-Bio^{®} or Perkin Elmer^{®} low and ultra-low attachment plates.

Alternatively, methods to prepare low attachment culture vessels, in particular low attachment plates, are well known to those skilled in the art. Such methods include coating untreated culture vessels with 1% agarose or coating untreated culture vessels with poly-2-hydroxyethyl methacrylate (also referred to as poly-HEMA).

In one embodiment, the PHH to be cultured with the method of the invention were stored frozen. Thus, in one embodiment, the PHH to be cultured with the method of the invention are first thawed.

Methods to thaw previously frozen animal cells are well-known in the field of animal cell culture.

In one embodiment, the PHH to be cultured with the method of the invention are first cultured in a low attachment plate (LAP) or in an ultra-low attachment plate (ULA) such as, for example, Corning^{®} Costar^{®}, InSphero^{®}, S-Bio^{®} or Perkin Elmer^{®} low or ultra-low attachment plates.

In one embodiment, the PHH are cultured in the non-adherent culture vessel as described above for at least about 1h, 2h, 3h, 4h, 5h, 6h, 7h, 8h, 9h, 10h, 11h, 12h, 13h, 14h, or 15h.

In one embodiment, the PHH are cultured in the non-adherent culture vessel as described above for at most about 96h, 84h, 72h, 60h, 48h, 36h, 24h, 23h, 22h, 21h, or 20h.

In one embodiment, the PHH are cultured in the non-adherent culture vessel as described above for a duration ranging from about 1h to about 96h, preferably from about 5h to about 48h, more preferably from about 10h to about 20h.

In one embodiment, the PHH are cultured in the non-adherent culture vessel as described above at a concentration of at least about 10², 5x10², or 10³ cells per cm².

In one embodiment, the PHH are cultured in the non-adherent culture vessel as described above at a concentration of at most about 10⁶, 5x10⁵ or 10⁵ cells per cm².

In one embodiment, the PHH are cultured in the non-adherent culture vessel as described above at a concentration ranging from about 10² to about 10⁶ cells per cm², preferably from about 10³ to about 10⁵ cells per cm², more preferably from about 1x10⁴ to about 9x10⁴ cells per cm².

In one embodiment, the PHH are cultured in the non-adherent culture vessel as described above, at a concentration ranging from about 10⁵ to about 5x10⁵ cells per cm², preferably from about 2x10⁵ to about 2.5x10⁵ cells per cm². In one embodiment, the PHH are cultured in a low or ultra-low attachment plate at a concentration ranging from about 10⁶ to about 5x10⁶ cells per well, preferably from about 2x10⁶ to about 2.5x10⁶ cells per well, said well preferably having a surface of about 10 cm².

In one embodiment, the PHH are cultured in the non-adherent culture vessel as described above at a concentration of about 10², 10³, 10⁴, 10⁵, or 10⁶ cells per cm².

In one embodiment, the PHH are cultured in the non-adherent culture vessel as described above at a concentration of about 1x10⁴, 2x10⁴, 3x10⁴, 4x10⁴, 5x10⁴, 6x10⁴, 7x10⁴, 8x10⁴, or 9x10⁴ cells per cm².

In one embodiment, the PHH are cultured in the non-adherent culture vessel as described above at a concentration of at least about 5x10³, 10⁴, or 5x10⁴ cells per ml of culture medium.

In one embodiment, the PHH are cultured in non-adherent culture vessel as described above at a concentration of at most about 2x10⁷, 10⁷, or 5x10⁶ cells per ml of culture medium.

In one embodiment, the PHH are cultured in the non-adherent culture vessel as described above at a concentration ranging from about 5x10³ to about 2x10⁷ cells per ml of culture medium, preferably from about 10⁴ to about 10⁷ cells per ml of culture medium, more preferably from about 10⁵ to about 10⁶ cells per ml of culture medium.

In one embodiment, the PHH are cultured in the non-adherent culture vessel as described above at a concentration of about 10⁴, 10⁵, 10⁶, or 10⁷ cells per ml of culture medium.

Selecting conditions suitable for the culture of animal cells is well known to those skilled in the art. Thus, the culture conditions to be used for culturing PHH in the non-adherent culture vessel as described above according to the method of the invention, such as, for example, culture medium, temperature, humidity and levels of CO₂, will be apparent to those having skill in the art.

Culture media that may be used according to the method of the invention include natural media and synthetic media, such as, for example, serum-containing media, serum-free media, xeno-free media notably for human cell culture, protein-free media, chemically defined media.

Examples of culture media include William's E medium, Basal Medium Eagle (BME), Eagle's Minimum Essential Medium (EMEM), Minimum Essential Medium (MEM), Dulbecco's Modified Eagles Medium (DMEM), Ham's F-10, Ham's F-12 medium, Kaighn's modified Ham's F-12 medium, DMEM/F-12 medium, and McCoy's 5A medium.

Culture media also include culture media suitable for the culture of hepatocytes (*e.g*., William's E Medium).

According to the present invention, the culture medium may be supplemented with additional substances such as salts, carbon sources, amino acids, serum and serum components, vitamins, minerals, reducing agents, buffering agents, lipids, nucleosides, antibiotics, cytokines, and growth factors.

According to one embodiment, the PHH to be cultured with the method of the invention are cultured in the non-adherent culture vessel as described above in a medium suitable for the culture of primary human cells.

Media suitable for the culture of primary animal cells are commercially available. The medium suitable for the culture of primary human cells will be apparent to those skilled in the art and will depend on the primary animal cells to be cultured (*e.g.*, hepatocytes).

According to one embodiment, the PHH to be cultured with the method of the invention are cultured in the non-adherent culture vessel as described above in a medium suitable for the culture of primary human hepatocytes.

Examples of media suitable for the culture of primary human hepatocytes, include Dulbecco's Modified Eagles Medium (DMEM), William's E Medium, Hepatocyte Culture Medium, Basal HepaRG Medium, HBM Basal Medium, and Hepatocyte Basal Medium.

According to the present invention, said media suitable for the culture of primary human hepatocytes may be supplemented, such as, for example, with L-glutamine, albumin, penicillin, streptomycin, insulin, transferrin, sodium pyruvate, sodium selenite, hydrocortisone or dexamethasone, HGF (hepatocyte growth factor), EGF (epidermal growth factor) and/or FCS (fetal calf serum) also referred to as FBS (fetal bovine serum).

In one embodiment, said media suitable for the culture of primary human hepatocytes may be supplemented, such as, for example, with gentamicin, L-glutamine, albumin, penicillin, streptomycin, insulin, transferrin, sodium pyruvate, sodium selenite, hydrocortisone or dexamethasone, HGF (hepatocyte growth factor), EGF (epidermal growth factor) and/or FCS (fetal calf serum) also referred to as FBS (fetal bovine serum).

In one embodiment, the PHH to be cultured according to the method of the invention are cultured in a low or ultra-low attachment culture vessel, preferably a low or ultra-low attachment plate, in William's E Medium supplemented with penicillin, streptomycin, insulin, glutamine (L-glutamine) and albumin, and optionally supplemented with transferrin, sodium selenite, hydrocortisone, HGF (hepatocyte growth factor), EGF (epidermal growth factor) and/or FCS (fetal calf serum), also referred to as WE HH (William's E medium human hepatocytes).

In one embodiment, the PHH to be cultured according to the method of the invention are cultured in a low or ultra-low attachment culture vessel, preferably a low or ultra-low attachment plate, in WE HH medium comprising penicillin, streptomycin, insulin, glutamine (L-glutamine), albumin and optionally FCS (fetal calf serum).

In one embodiment, the WE HH medium comprises penicillin at a concentration ranging from about 50 to about 200 U/mL, preferably about 100 U/mL; streptomycin at a concentration ranging from about 50 to about 200 µg/mL, preferably 100 µg/mL; insulin at a concentration ranging from about 5 to about 30 µg/mL, preferably about 5 µg/mL or about 15 µg/mL; glutamine (L-glutamine) at a concentration ranging from about 1 to about 10 mM, preferably about 2 mM; albumin at a concentration ranging from about 0.01 to about 1% (w/v), preferably about 0.1% (w/v); and FCS (fetal calf serum) at a concentration ranging from about 0 to about 20% (v/v), or from 1% to about 20% (v/v), and preferably about 10% (v/v).

In one embodiment, the PHH to be cultured according to the method of the invention are cultured in a low or ultra-low attachment culture vessel, preferably a low or ultra-low attachment plate, in a culture medium, preferably in William's E Medium, which does not comprise or is not supplemented with FCS (fetal calf serum).

In one embodiment, the PHH to be cultured according to the method of the invention are cultured in a low or ultra-low attachment culture vessel, preferably a low or ultra-low attachment plate, in WE HH medium supplemented with penicillin, streptomycin, insulin, glutamine (L-glutamine), albumin and hydrocortisone and optionally supplemented with transferrin, sodium selenite, HGF (hepatocyte growth factor), EGF (epidermal growth factor) and/or FCS (fetal calf serum).

In one embodiment, the WE HH medium is supplemented with penicillin, streptomycin, insulin, glutamine (L-glutamine) and albumin, with or without FCS (fetal calf serum).

In one embodiment, the WE HH medium is supplemented with penicillin, streptomycin, insulin, glutamine (L-glutamine), albumin, hydrocortisone, transferrin, sodium selenite, HGF (hepatocyte growth factor) and EGF (epidermal growth factor), with or without FCS (fetal calf serum).

In one embodiment, the WE HH medium comprises insulin at a concentration ranging from about 5 to about 30 µg/mL, preferably ranging from about 5 to about 15 µg/mL, more preferably about 5 µg/mL or about 15 µg/mL; transferrin at a concentration ranging from about 0 to about 10 µg/mL, or from about 0.1 to about 10 µg/mL, and preferably about 5.5 µg/mL; sodium selenite at a concentration ranging from about 0 to about 10 µg/mL, or from about 0.1 to about 10 µg/mL, and preferably about 5 µg/mL; hydrocortisone at a concentration ranging from about 0.1 to about 50 µM, preferably about 1 µM; rhHGF (recombinant human hepatocyte growth factor) at a concentration ranging from about 0 to about 10 ng/mL, or from about 0.1 to about 10 ng/mL, and preferably about 2.5 ng/mL; rhEGF (recombinant human epidermal growth factor) at a concentration ranging from about 0 to about 1 ng/µL, or from about 0.01 to about 1 ng/µL, and preferably about 0.05 ng/µL; FCS (fetal calf serum) at a concentration ranging from about 0 to about 20% (v/v), or from about 1% to about 20% (v/v), and preferably about 10% (v/v); glutamine (L-glutamine) at a concentration ranging from about 1 to about 10 mM, preferably about 2 mM; albumin at a concentration ranging from about 0.01 to about 1% (w/v), preferably about 0.1% (w/v); penicillin at a concentration ranging from about 50 to about 200 U/mL, preferably about 100 U/mL; streptomycin at a concentration ranging from about 50 to about 200 µg/mL, preferably 100 µg/mL.

In one embodiment, the WE HH medium as described hereinabove further comprises gentamicin.

Thus, in one embodiment, the PHH to be cultured according to the method of the invention are cultured in a low or ultra-low attachment culture vessel, preferably a low or ultra-low attachment plate, in WE HH medium supplemented with gentamicin, penicillin, streptomycin, insulin, glutamine (L-glutamine), albumin and hydrocortisone, with or without FCS (fetal calf serum).

In one embodiment, the WE HH medium as described hereinabove comprises gentamicin at a concentration ranging from about 1 to about 100 µg/mL, preferably about 50 µg/mL.

Thus, in one embodiment, the PHH to be cultured according to the method of the invention are cultured in a low or ultra-low attachment culture vessel, preferably a low or ultra-low attachment plate, in WE HH medium supplemented with gentamicin at a concentration ranging from about 1 to about 100 µg/mL, preferably about 50 µg/mL; penicillin at a concentration ranging from about 50 to about 200 U/mL, preferably about 100 U/mL; streptomycin at a concentration ranging from about 50 to about 200 µg/mL, preferably 100 µg/mL; insulin at a concentration ranging from about 5 to about 30 µg/mL, preferably ranging from about 5 to about 15 µg/mL, more preferably about 5 µg/mL; glutamine (L-glutamine) at a concentration ranging from about 1 to about 10 mM, preferably about 2 mM; albumin at a concentration ranging from about 0.01 to about 1% (w/v), preferably about 0.1% (w/v); with or without FCS (fetal calf serum) at a concentration ranging from about 1% to about 20% (v/v), and preferably about 10% (v/v).

In one embodiment, the PHH to be cultured according to the method of the invention are cultured in the non-adherent culture vessel, preferably a low or ultra-low attachment culture vessel, at about 37°C and about 5% CO₂.

According to the method of the invention, culturing the PHH in the non-adherent culture vessel as described above allows to obtain aggregates of said PHH.

In one embodiment, after culturing the PHH in the non-adherent culture vessel as described above at least about 30%, 35%, 40%, 45%, 50%, 55%, 60%, 65%, 70% ,75% or 80%, preferably at least about 50%, of the PHH are aggregated.

Methods to observe the formation of cell aggregates are well-known to one skilled in the art and include, for example, fluorescence microscopy such as two-photon excited fluorescence (TPEF) microscopy.

According to the method of the invention, after being first cultured in a non-adherent culture vessel as described above, the PHH are transferred to a culture medium comprising collagen or truncated collagen, *i.e.,* gelatin, and are thus embedded in a collagen or gelatin matrix. Without wishing to be bound by any theory, the Applicant suggests that embedding primary human hepatocytes (PHH) in a collagen matrix or a gelatin matrix, in particular a methacrylated gelatin (GelMa) matrix, provides the PHH with a microenvironment enabling the proliferation of the PHH and the formation of PHH spheroids according to the invention, *i.e.,* spheres delimited by a single layer of well-organized PHH forming acinus-like structures with a hollow lumen.

The Applicant indeed showed that continuously culturing the PHH in a non-adherent culture vessel as described above without ever embedding the PHH in a collagen or gelatin matrix did not allow to obtain spheroids comprising proliferative PHH. In particular, the Applicant showed that primary human hepatocytes first cultured in a non-adherent culture vessel such as a low attachment plate must be transferred to a culture medium comprising collagen or gelatin, in particular methacrylated gelatin (GelMa), and embedded in a collagen matrix or in a gelatin matrix, in particular a GelMa matrix, in order to obtain spheroids comprising proliferative primary human hepatocytes, wherein said spheroids are acinus-like structures with a hollow lumen, delimited by a single layer of well-organized PHH forming.

According to the present invention, collagen is added to a culture medium as described hereinabove in order to obtain a culture medium comprising collagen, and thus to transfer the PHH first cultured in a non-adherent culture vessel in said culture medium comprising collagen.

As used herein, the term "collagen" encompasses the different sub-families of collagens and truncated collagen also referred to as gelatin.

Thus, in one embodiment, the PHH are transferred to a culture medium comprising truncated collagen, *i.e*., gelatin.

In one embodiment, the PHH are transferred to a culture medium comprising fibrillar collagen.

In one embodiment, the PHH are transferred to a culture medium comprising gelatin derived from fibrillar collagen.

Examples of fibrillar collagen include type I collagen, type II collagen, type III collagen, type V collagen, type VI collagen, type XI collagen, type XXIV collagen and type XXVII collagen.

In one embodiment, the PHH are transferred to a culture medium comprising fibrillar collagen selected from the group comprising or consisting of type I collagen, type II collagen, type III collagen, type V collagen, type VI collagen, type XI collagen, type XXIV collagen, type XXVII collagen and any mixtures thereof.

In one embodiment, the PHH are transferred to a culture medium comprising gelatin derived from fibrillar collagen selected from the group comprising or consisting of type I collagen, type II collagen, type III collagen, type V collagen, type VI collagen, type XI collagen, type XXIV collagen, type XXVII collagen and any mixtures thereof.

In one embodiment, the PHH are transferred to a culture medium comprising fibrillar collagen selected from the group comprising or consisting of type I collagen, type III collagen, and any mixtures thereof.

In one embodiment, the PHH are transferred to a culture medium comprising gelatin derived from fibrillar collagen selected from the group comprising or consisting of type I collagen, type III collagen, and any mixtures thereof.

In one embodiment, the PHH are transferred to a culture medium comprising type I collagen.

In one embodiment, the PHH are transferred to a culture medium comprising gelatin derived from type I collagen. In one embodiment, the PHH are transferred to a culture medium comprising type A gelatin, in particular type A gelatin derived from type I collagen.

Examples of type I collagen that may be used according to the method of the invention include rat type I collagen, bovine type I collagen, porcine type I collagen, human type I collagen from human placenta, recombinant human type I collagen, type I collagen from rabbit skin, ovine type I collagen and type I-related fibrillar collagen from jelly fish and sea materials.

In one embodiment, the PHH are transferred to a culture medium comprising collagen as described hereinabove. According to the present invention, the culture medium comprising collagen as described hereinabove comprises collagen at a concentration ranging from about 0.75 mg/mL to about 3 mg/mL.

In one embodiment, the PHH are transferred to a culture medium comprising collagen as described hereinabove at a concentration of at most about 2.75 mg/mL, 2.5mg/mL, 2.25 mg/mL or 2 mg/mL.. In another embodiment, the PHH are transferred to a culture medium comprising collagen as described hereinabove at a concentration of at most about 2.9 mg/mL, 2.8 mg/mL, 2.7 mg/mL, 2.6 mg/mL, 2.5 mg/mL, 2.4 mg/mL, 2.3 mg/mL, 2.2 mg/mL, 2.1 mg/mL or 2 mg/mL.

In one embodiment, the PHH are transferred to a culture medium comprising collagen as described hereinabove at a concentration ranging from about 0.75 mg/mL to about 1.5 mg/mL.

In one embodiment, the PHH are transferred to a culture medium comprising collagen at a concentration of about 0.75 mg/mL, 1 mg/mL, 1.25 mg/mL or 1.5 mg/mL.

In one embodiment, the PHH are transferred to a culture medium comprising gelatin as described hereinabove. According to the present invention, the culture medium comprising gelatin as described hereinabove comprises gelatin at a concentration ranging from about 15% (w/v) to about 40% (w/v). In one embodiment, the PHH are transferred to a culture medium comprising gelatin as described hereinabove at a concentration of at least about 20% (v/w), 25% (w/v), 30% (w/v), or 35% (w/v). Thus, in one embodiment, the PHH are transferred to a culture medium comprising gelatin as described hereinabove at a concentration of at least about 200 mg/mL, 250 mg/mL, 300 mg/mL, or 350 mg/mL.

In a particular embodiment, the PHH are transferred to a culture medium comprising methacrylated gelatin (GelMa).

In one embodiment, GelMa is obtained by the methacrylation of gelatin as described hereinabove with methacrylic anhydride. In one embodiment, GelMa is obtained by the methacrylation of gelatin derived from type I collagen with methacrylic anhydride. In one embodiment, GelMa is obtained by the methacrylation of type A gelatin. In one embodiment, GelMa is obtained by the methacrylation of type A gelatin derived from type I collagen with methacrylic anhydride.

According to the present invention, the culture medium comprising GelMa as described hereinabove comprises GelMa at a concentration ranging from about 1% (w/v) to about 10% (w/v). In one embodiment, the PHH are transferred to a culture medium comprising GelMa as described hereinabove at a concentration of at least about 2% (w/v), 2.5% (w/v), 3% (w/v), 4% (w/v) or 5% (w/v). Thus, in one embodiment, the PHH are transferred to a culture medium comprising GelMa as described hereinabove at a concentration of at least about 20 mg/mL, 25 mg/mL, 30 mg/mL, 40 mg/mL or 50 mg/mL.

In one embodiment, the PHH are transferred to a culture medium comprising GelMa as described hereinabove at a concentration of at most about 9% (w/v), 8% (w/v), 7% (w/v), 7.5 % (w/v), 6 % (w/v) or 5% (w/v). Thus, in one embodiment, the PHH are transferred to a culture medium comprising GelMa as described hereinabove at a concentration of at most about 90 mg/mL, 80 mg/mL, 70 mg/mL, 75 mg/mL, 60 mg/mL or 50 mg/mL.

In one embodiment, the PHH are transferred to a culture medium comprising GelMa as described hereinabove at a concentration ranging from about 2.5% (w/v) to about 10% (w/v), preferably from about 4% (w/v) to about 6% (w/v). Thus, in one embodiment, the PHH are transferred to a culture medium comprising GelMa as described hereinabove at a concentration ranging from about 25 mg/mL to about 100 mg/mL, preferably from about 40 mg/mL to about 60 mg/mL.

In one embodiment, the PHH are transferred to a culture medium comprising GelMa at a concentration of about 5 % (w/v), *i.e.,* at a concentration of about 50 mg/mL.

In one embodiment, the concentration of collagen, truncated collagen (*i.e.,* gelatin) or methacrylated gelatin comprised in the culture medium as described hereinabove is referred to as the final concentration of collagen, truncated collagen (*i.e*., gelatin) or methacrylated gelatin in the culture medium.

In one embodiment, the PHH are transferred to a culture medium comprising GelMa as described hereinabove and further comprising a photoinitiator to induce polymerization upon light exposure.

Examples of photoinitiators include 2,2'-azobis[2-methyl-n-(2-hydroxyethyl)propionamide] (also known as VA-086), 1-[4-(2-hydroxyethoxy)-phenyl]-2-hydroxy-2-methyl-1-propanon (also known as Irgacure 2959), lithium phenyl-2,4,6-trimethylbenzoylphosphinate (also known as LAP or BioKey), 2',4',5',7'-tetrabromofluorescein disodium salt (also known as eosin Y or EY).

In one embodiment, the photoinitiator is selected from the group comprising or consisting of 2,2'-azobis[2-methyl-n-(2-hydroxyethyl)propionamide] (also known as VA-086), 1-[4-(2-hydroxyethoxy)-phenyl]-2-hydroxy-2-methyl-1-propanon (also known as Irgacure 2959), lithium phenyl-2,4,6-trimethylbenzoylphosphinate (also known as LAP or BioKey), and 2',4',5',7'-tetrabromofluorescein disodium salt (also known as eosin Y or EY), preferably the photoinitiator is lithium phenyl-2,4,6-trimethylbenzoylphosphinate (also known as LAP or BioKey).

In one embodiment, the PHH are transferred to a culture medium comprising GelMa as described hereinabove and further comprising a photoinitiator as described hereinabove at a concentration of at least about 0.01% (w/v), 0.05% (w/v), 0.075% (w/v), 0.1% (w/v), 0.25% (w/v), or 0.5% (w/v). Thus, in one embodiment, the PHH are transferred to a culture medium comprising GelMa as described hereinabove and further comprising a photoinitiator as described hereinabove at a concentration of at least about 0.1 mg/mL, 0.5 mg/mL, 0.75 mg/mL, 1 mg/mL, 2.5 mg/mL or 5 mg/mL.

In one embodiment, the PHH are transferred to a culture medium comprising GelMa as described hereinabove and further comprising a photoinitiator as described hereinabove at a concentration of at most about 2% (w/v), 1.5% (w/v), 1% (w/v), 0.75% (w/v), 0.5% (w/v), 0.25% (w/v), 0.1% (w/v) or 0,05% (w/v). Thus, in one embodiment, the PHH are transferred to a culture medium comprising GelMa as described hereinabove and further comprising a photoinitiator as described hereinabove at a concentration of at most about 20 mg/mL, 15 mg/mL, 10 mg/mL, 7.5 mg/mL, 5 mg/mL, 2.5 mg/mL, 1 mg/mL or 0,5 mg/mL.

In one embodiment, the PHH are transferred to a culture medium comprising GelMa as described hereinabove and further comprising a photoinitiator as described hereinabove at a concentration ranging from about 0.01% (w/v) to about 2% (w/v). Thus, in one embodiment, the PHH are transferred to a culture medium comprising GelMa as described hereinabove and further comprising a photoinitiator as described hereinabove at a concentration ranging from about 0.1 mg/mL to about 20 mg/mL.

In one embodiment, the PHH are transferred to a culture medium comprising GelMa as described hereinabove and further comprising a photoinitiator as described hereinabove at a concentration of about 1% (w/v), *i.e.,* at a concentration of about 1 mg/mL.

In one embodiment, the culture medium in which collagen or gelatin is added to obtain a culture medium comprising collagen or gelatin and the culture medium previously used to culture the PHH in a non-adherent culture vessel as described hereinabove are different media. In another embodiment, the culture medium in which collagen or gelatin is added to obtain a culture medium comprising collagen or gelatin and the culture medium previously used to culture the PHH in a non-adherent culture vessel as described hereinabove are the same media.

As mentioned hereinabove, the culture medium to be used according to the method of the invention will be apparent to those skilled in the art. Culture media that may be used according to the method of the invention are described hereinabove.

According to one embodiment, the PHH to be cultured with the method of the invention are transferred to a medium suitable for the culture of primary human hepatocytes, said culture medium further comprising collagen or gelatin as described hereinabove.

Examples of media suitable for the culture of primary human hepatocytes are listed hereinabove.

In one embodiment, the PHH to be cultured according to the method of the invention are transferred in WE HH as defined hereinabove, said WE HH further comprising collagen or gelatin as described hereinabove. In one embodiment, the PHH to be cultured according to the method of the invention are transferred in WE HH as defined hereinabove, said WE HH further comprising methacrylated gelatin as described hereinabove. In one embodiment, the PHH to be cultured according to the method of the invention are transferred in WE HH as defined hereinabove, said WE HH further comprising methacrylated gelatin and a photoinitiator, preferably lithium phenyl-2,4,6-trimethylbenzoylphosphinate, as described hereinabove.

In one embodiment, the WE HH medium is supplemented with penicillin, streptomycin, insulin, glutamine (L-glutamine), albumin, transferrin, sodium selenite, hydrocortisone, HGF (hepatocyte growth factor) and EGF (epidermal growth factor), and optionally gentamicin and/or FCS (fetal calf serum).

In one embodiment, the WE HH medium comprises penicillin at a concentration ranging from about 50 to about 200 U/mL, preferably about 100 U/mL; streptomycin at a concentration ranging from about 50 to about 200 µg/mL, preferably 100 µg/mL; insulin at a concentration ranging from about 5 to about 30 µg/mL, preferably ranging from about 5 to about 15 µg/mL, preferably about 15 µg/mL; glutamine (L-glutamine) at a concentration ranging from about 1 to about 10 mM, preferably about 2 mM; albumin at a concentration ranging from about 0.01 to about 1% (w/v), preferably about 0.1% (w/v); transferrin at a concentration ranging from about 0 to about 10 µg/mL, or from about 0.1 to about 10 µg/mL, and preferably about 5.5 µg/mL; sodium selenite at a concentration ranging from about 0 to about 10 µg/mL, or from about 0.1 to about 10 µg/mL, and preferably about 5 µg/mL; hydrocortisone at a concentration ranging from about 0.1 to about 50 µM, preferably about 1 µM; rhHGF (recombinant human hepatocyte growth factor) at a concentration ranging from about 0 to about 10 ng/mL, or from about 0.1 to about 10 ng/mL, and preferably about 2.5 ng/mL; rhEGF (recombinant human epidermal growth factor) at a concentration ranging from about 0 to about 1 ng/µL, or from about 0.01 to about 1 ng/µL, and preferably about 0.05 ng/µL; and FCS (fetal calf serum) at a concentration ranging from about 0 to about 20% (v/v), or from about 1% to about 20% (v/v), and preferably about 10% (v/v); and optionally gentamicin at a concentration ranging from about 1 to about 100 µg/mL, preferably about 50 µg/mL.

In one embodiment, the PHH are transferred to a culture medium comprising collagen or gelatin as described hereinabove at a concentration of at least about 10³, 5.10³, or 10⁴ cells/mL of culture medium comprising collagen or gelatin.

In one embodiment, the PHH are transferred to a culture medium comprising collagen or gelatin as described hereinabove at a concentration of at most about 10⁷, 5x10⁶ or 10⁶ cells/mL of culture medium comprising collagen or gelatin.

In one embodiment, the PHH are transferred to a culture medium comprising collagen or gelatin as described hereinabove at a concentration ranging from about 10³ cells/mL to about 10⁷ cells/mL, preferably from about 10⁴ cells/mL to about 10⁶ cells/mL, more preferably from about 1x10⁵ to about 9x10⁵ cells/mL of culture medium comprising collagen or gelatin.

In another embodiment, the PHH are transferred to a culture medium comprising collagen or gelatin as described hereinabove at a concentration ranging from about 3x10⁵ cells/mL to about 4.5x10⁵ cells/mL, preferably from about 3.25x10⁵ cells/mL to about 4x10⁵ cells/mL, more preferably from about 3.5x10⁵ cells/mL to about 3.75x10⁵ cells/mL of culture medium comprising collagen or gelatin.

In one embodiment, the PHH are transferred to a culture medium comprising collagen or gelatin as described hereinabove at a concentration of about 3x10⁵, 3.25x10⁵, 3.5x10⁵ 3.65x10⁵, 3.75x10⁵, 4x10⁵, 4.25x10⁵ or 4.5 x10⁵ cells/mL of culture medium comprising collagen or gelatin. In another embodiment, the PHH are transferred to a culture medium comprising collagen as described hereinabove at a concentration of about 3.65x10⁵ cells/mL of culture medium comprising collagen or gelatin.

In one embodiment, after transfer of the PHH to a culture medium comprising collagen or gelatin as described hereinabove, the pH of the resulting mix is adjusted at a value ranging from about 7 to about 8, preferably at a value of about 7.4. Methods to adjust the pH of a culture medium are well-known to the person skilled in the art. For example, the pH of a culture medium may be increased as required with the addition of an appropriate volume of a NaOH solution. Alternatively, the pH of a culture medium may be lowered as required with the addition of an appropriate volume of a HCl solution.

Selecting conditions suitable for the culture of animal cells is well known to those skilled in the art. Thus, the culture conditions to be used according to the method of the invention, such as, for example, culture vessel, temperature, humidity and levels of CO₂, will be apparent to those having skill in the art.

In one embodiment, the mix resulting from the transfer of PHH to a culture medium comprising collagen or gelatin as described hereinabove is poured into a culture vessel such as a plate or a multi-well plate, for example, a 24-well plate, a 48-well plate or a 96-well plate. It will be evident to those skilled in the art that the volume poured in the culture vessel will depend on the size of the culture vessel, for example on the size of the plate. In the case of a multi-well plate, the volume poured per well will depend on the size of the well and thus on the size of the multi-well plate.

Thus, in one embodiment, about 100µL of the mix resulting from the transfer of PHH to a culture medium comprising collagen or gelatin as described hereinabove are poured per well of a 96-well plate, about 300µL are poured per well of a 48-well plate, and about 400µL are poured per well of a 24-well plate.

According to one embodiment of the method of the invention, the mix resulting from the transfer of PHH to a culture medium comprising collagen or gelatin as described hereinabove is incubated in a culture vessel, preferably a multi-well plate, for at least about 10 min, 20 min, 30 min, 45 min, 1h, 2h, 3h, or 4h, preferably for at least about 2h.

In one embodiment, the mix resulting from the transfer of primary human hepatocytes to a culture medium comprising collagen is incubated as described hereinabove at about 37°C and about 5% CO₂, humidity 80-100%, preferably 85-95%.

According to one embodiment of the method of the invention, after an incubation at 37°C of at least about 10 min, 20 min, 30 min, 45 min, 1h, 2h, 3h, or 4h, preferably of at least about 2h, the collagen comprised in the culture medium as described hereinabove is polymerized and the PHH are thus embedded in a collagen matrix.

According to another embodiment of the method of the invention, the mix resulting from the transfer of PHH to a culture medium comprising methacrylated gelatin (GelMa) as described hereinabove is illuminated with light having a wavelength ranging from about 250 nm to about 530 nm, preferably from about 365 to about 405 nm. In one embodiment, the mix resulting from the transfer of PHH to a culture medium comprising methacrylated gelatin (GelMa) as described hereinabove is illuminated with light having a wavelength of about 365 nm or 405 nm.

In one embodiment, the mix resulting from the transfer of PHH to a culture medium comprising methacrylated gelatin (GelMa) as described hereinabove is illuminated as described hereinabove for a time ranging from about 10 seconds to about 10 minutes, preferably from about 30 seconds to about 5 minutes.

It is well-known in the field that the time of illumination will depend on the wavelength and on the intensity of the light. Accordingly, the intensity of the light will depend on the wavelength of the light and on the time of illumination.

Examples of light intensity include intensities ranging from about 1 mW/cm² to about 150 mW/cm².

According to the method of the invention, after illumination, preferably with a light having a wavelength of about 365 nm or 405 nm, for a time of about 60 seconds, the methacrylated gelatin (GelMa) comprised in the culture medium as described hereinabove is polymerized and the PHH are thus embedded in a GelMa matrix.

The method of the invention comprises a third step of culturing the PHH embedded in the collagen or gelatin matrix as described hereinabove and thus allows to obtain spheroids comprising proliferative PHH.

According to the method of the invention, culture medium is added to the culture vessel comprising the collagen or gelatin matrix as described hereinabove.

In one embodiment culture medium is added to the culture vessel comprising the collagen or gelatin matrix as described hereinabove in a ratio ranging from about 1.5:1 to about 1:1.5 with respect to the previously added volume of the mix resulting from the transfer of PHH to a culture medium comprising collagen or gelatin as described hereinabove. In one embodiment culture medium is added to the culture vessel comprising the collagen or gelatin matrix as described hereinabove in a ratio of about 1.5:1, 1.4:1, 1.3:1, 1.2:1, 1.1:1, 1:1, 1:1.1, 1:1.2, 1:1.3, 1:1.4, or 1:1.5, preferably in a ratio of about 1:1, with respect to the previously added volume of the mix resulting from the transfer of PHH to a culture medium comprising collagen or gelatin as described hereinabove.

In one embodiment, the culture medium added as described hereinabove and the culture medium used to obtain the collagen or gelatin matrix of the invention are different media. In another embodiment, the culture medium added as described hereinabove and the culture medium used to obtain the collagen or gelatin matrix of the invention are the same media. Preferably, the same culture medium is used in the second step and in the third step of the method of the invention.

As mentioned hereinabove, the culture medium to be used according to the method of the invention will be apparent to those skilled in the art. Culture media that may be used according to the method of the invention are described hereinabove.

According to one embodiment, the PHH embedded in the collagen or gelatin matrix according to the invention are cultured in a medium suitable for the culture of primary human hepatocytes.

Examples of media suitable for the culture of primary human hepatocytes are listed hereinabove.

In one embodiment, primary human hepatocytes are embedded in the collagen matrix or in the gelatin matrix, in particular the GelMa matrix, according to the invention and are cultured in WE HH as defined hereinabove.

In one embodiment, the WE HH medium is supplemented with penicillin, streptomycin, insulin, glutamine (L-glutamine), albumin, transferrin, sodium selenite, hydrocortisone, HGF (hepatocyte growth factor) and EGF (epidermal growth factor), and optionally gentamicin and/or FCS (fetal calf serum).

In one embodiment, the WE HH medium comprises penicillin at a concentration ranging from about 50 to about 200 U/mL, preferably about 100 U/mL; streptomycin at a concentration ranging from about 50 to about 200 µg/mL, preferably 100 µg/mL; insulin at a concentration ranging from about 5 to about 30 µg/mL, preferably ranging from about 5 to about 15 µg/mL, preferably about 15 µg/mL; glutamine (L-glutamine) at a concentration ranging from about 1 to about 10 mM, preferably about 2 mM; albumin at a concentration ranging from about 0.01 to about 1% (w/v), preferably about 0.1% (w/v); transferrin at a concentration ranging from about 0 to about 10 µg/mL, or from about 0.1 to about 10 µg/mL, and preferably about 5.5 µg/mL; sodium selenite at a concentration ranging from about 0 to about 10 µg/mL, or from about 0.1 to about 10 µg/mL, and preferably about 5 µg/mL; hydrocortisone at a concentration ranging from about 0.1 to about 50 µM, preferably about 1 µM; rhHGF (recombinant human hepatocyte growth factor) at a concentration ranging from about 0 to about 10 ng/mL, or from about 0.1 to about 10 ng/mL, and preferably about 2.5 ng/mL; rhEGF (recombinant human epidermal growth factor) at a concentration ranging from about 0 to about 1 ng/µL, or from about 0.01 to about 1 ng/µL, and preferably about 0.05 ng/ µL; and FCS (fetal calf serum) at a concentration ranging from about 0 to about 20% (v/v), or from about 1% to about 20%

(v/v), and preferably about 10% (v/v); and optionally gentamicin at a concentration ranging from about 1 to about 100 µg/mL, preferably about 50 µg/mL.

Selecting conditions suitable for the culture of animal cells is well known to those skilled in the art. Thus, the culture conditions to be used for culturing PHH embedded in the collagen or gelatin matrix according to the method of the invention, such as, for example, medium renewal, temperature, humidity and levels of CO₂ will be apparent to those having skill in the art.

In one embodiment, the culture medium added to the culture vessel comprising the collagen or gelatin matrix as described hereinabove is changed every 1, 2, 3, 4 or more days, preferably every 2 days.

In one embodiment, the culture medium added to the culture vessel comprising the collagen or gelatin matrix as described hereinabove is changed every 24h, 36h, 48h, 60h, 72h or more, preferably every 48h.

The frequency at which to change the culture medium will be apparent to those skilled in the art and will depend on the purpose of the culture.

In one embodiment, the PHH embedded in the collagen or gelatin matrix according to the method of the invention are cultured at about 37°C and about 5% CO₂, humidity 80-100%, preferably 85-95%.

In one embodiment, the PHH embedded in the collagen or gelatin matrix according to the method of the invention are cultured for at least about 1, 2, 3, 4, 5, 6, 7, 8, 9, 10, 11, 12, 13, 14, 15, 20, 25, 30, or 35 days.

In one embodiment, the PHH embedded in the collagen or gelatin matrix according to the method of the invention are cultured for at least about 1, 2, 3, 4, 5, or 6 weeks.

According to one embodiment, the PHH embedded in the collagen matrix or in the gelatin matrix, in particular the GelMa matrix, according to the invention are cultured for at least about 2 days in order to obtain spheroids comprising proliferative PHH.

According to one embodiment, primary human hepatocytes are proliferative if at least about 10%, 15%, 20%, 25%, 30%, 35%, 40%, 45%, 50%, 55%, 60%, 65%, 70%, 75%, or 80%, preferably at least about 20%, more preferably at least about 30%, even more preferably at least about 40%, of the total number of primary human hepatocytes are positive for a proliferation marker such as, for example, positive for BrdU incorporation (BrdU⁺), positive for EdU incorporation (EdU⁺), positive for Ki67 expression (Ki67⁺), or positive for cyclin D1 expression (cyclin D1⁺).

In one embodiment, the total number of primary human hepatocytes is assessed through the detection of a hepatocyte marker, such as, for example, albumin (alb) expression.

In one embodiment, primary human hepatocytes are proliferative if at least about 10%, 15%, 20%, 25%, 30%, 35%, 40%, or 45%, preferably at least about 20%, more preferably at least about 30%, even more preferably at least about 40%, of the total number of Alb⁺ primary hepatocytes are positive for a proliferation marker such as, for example, positive for BrdU incorporation (BrdU⁺), positive for EdU incorporation (EdU⁺), positive for Ki67 expression (Ki67⁺), or positive for cyclin D1 expression (cyclin D1⁺).

In one embodiment, the total number of primary hepatocytes, in particular of Alb⁺ primary hepatocytes, correspond to the total number of primary hepatocytes, in particular of Alb⁺ primary hepatocytes observed or considered when assessing a proliferation marker, for example through the measurement of mRNA expressions or protein expressions and/or levels.

In one embodiment, the method of the invention further comprises a step to induce one or more additional wave(s) of proliferation of the PHH in culture in the collagen or gelatin matrix as described hereinabove.

In one embodiment, the method of the invention further comprises a step of transiently inhibiting the MAPK MEK1/2-ERK1/2 pathway in the PHH in culture in the collagen or gelatin matrix as described hereinabove.

The Applicant indeed showed that transiently inhibiting the MAPK MEK1/2-ERK1/2 pathway in PHH in culture in the collagen matrix as described hereinabove induced an additional wave of proliferation of said PHH. TheApplicant showed that primary human hepatocytes underwent an additional wave of proliferation induced by the addition of an inhibitor of the MAPK MEK1/2-ERK1/2 pathway to the culture.

As used in the present invention, inhibitors of the MAPK MEK1/2-ERK1/2 pathway encompass MEK1/2 inhibitors, MEK1 inhibitors, MEK2 inhibitors, ERK1/2 inhibitors, ERK1 inhibitors, and ERK2 inhibitors.

Thus, in one embodiment, the method of the invention further comprises a fourth step (*i.e.,* step d)) of adding a MEK1/2 inhibitor, a MEK1 inhibitor, a MEK2 inhibitor, an ERK1/2 inhibitor, an ERK1 inhibitor, or an ERK2 inhibitor to the culture of PHH embedded in the collagen matrix or in the gelatin matrix, in particular the GelMa matrix, as described hereinabove.

Examples of MEK inhibitors include U0126, PD98059, PD184352, CL-1040, FR180204, and BUD-523.

In one embodiment, the inhibitor of the MAPK MEK1/2-ERK1/2 pathway as described above is added to the culture of PHH embedded in the collagen or gelatin matrix at least 7, 8, 9, 10, 11, 12, 13, 14, 15, 16, 17, 18, 19, 20 or more days after the start of the culture of the PHH embedded in a collagen or gelatin matrix according to the invention.

In one embodiment, the inhibitor of the MAPK MEK1/2-ERK1/2 pathway as described above is added to the culture of PHH embedded in the collagen or gelatin matrix as described hereinabove for about 24h, 36h, 48h, 60h, or 72h, preferably for about 48h.

Thus, in one embodiment, the culture medium comprising the inhibitor of the MAPK MEK1/2-ERK1/2 pathway as described above is replaced with culture medium without MAPK MEK1/2-ERK1/2 pathway inhibitor after about 24h, 36h, 48h, 60h, or 72h, preferably after about 48h.

In one embodiment, the addition of an inhibitor of the MAPK MEK 1/2-ERK 1/2 pathway as described above is repeated to induce additional wave(s) of proliferation when desired.

In one embodiment, the spheroids comprising proliferative PHH obtained according to the method of the invention are embedded in the collagen matrix or in the gelatin matrix, in particular the GelMa matrix.

In one embodiment, the method of the invention further comprises a step to isolate the spheroids comprising proliferative PHH from the collagen matrix or from the gelatin matrix, in particular the GelMa matrix.

In one embodiment, the spheroids comprising proliferative PHH are isolated from the collagen or gelatin matrix after enzymatic digestion of the collagen. Thus, in one embodiment, the spheroids comprising proliferative PHH are isolated from the collagen or gelatin matrix after incubation with a collagenase or an enzyme mixture with collagenolytic activity.

In one embodiment, the method of the invention further comprises a final step (*e.g.,* step e)) of incubating the spheroids comprising proliferative PHH embedded in the collagen matrix or in the gelatin matrix, in particular the GelMa matrix, with a collagenase or an enzyme mixture with collagenolytic activity.

Examples of enzyme mixtures with collagenolytic activity include Liberase^{®} and Accutase^{®}.

The present invention thus relates to a method of culturing PHH, comprising:
a) first culturing the PHH in a non-adherent culture vessel as described hereinabove;
b) then transferring the PHH to a culture medium comprising collagen or truncated collagen, namely gelatin, thereby embedding the PHH in a collagen or gelatin matrix;
c) culturing the PHH embedded in the collagen or gelatin matrix, thereby obtaining spheroids comprising proliferative PHH;
d) optionally adding an inhibitor of the MAPK MEK1/2-ERK1/2 pathway as described above to the culture of PHH embedded in the collagen or gelatin matrix, thereby inducing one or more additional wave(s) of proliferation of the PHH; and
e) optionally incubating the spheroids comprising proliferative PHH embedded in the collagen or gelatin matrix with a collagenase or an enzyme mixture with collagenolytic activity, thereby isolating the spheroids comprising proliferative PHH from the collagen or gelatin matrix.

The present invention also relates to a method of culturing PHH, comprising:
a) first culturing the PHH in a non-adherent culture vessel as described hereinabove;
b) then transferring the PHH to a culture medium comprising collagen, preferably fibrillar collagen, thereby embedding the PHH in a collagen matrix, preferably a fibrillar collagen matrix;
c) culturing the PHH embedded in the collagen matrix, thereby obtaining spheroids comprising proliferative PHH;
d) optionally adding an inhibitor of the MAPK MEK1/2-ERK1/2 pathway as described above to the culture of PHH embedded in the collagen matrix, thereby inducing one or more additional wave(s) of proliferation of the PHH; and
e) optionally incubating the spheroids comprising proliferative PHH embedded in the collagen matrix with a collagenase or an enzyme mixture with collagenolytic activity, thereby isolating the spheroids comprising proliferative PHH from the collagen matrix.

In one embodiment, the present invention relates to a method of culturing PHH, comprising:
a) first culturing the PHH in a low or ultra-low attachment culture vessel, preferably a low or ultra-low attachment plate;
b) then transferring the PHH to a culture medium comprising fibrillar collagen, preferably type I collagen, at a concentration ranging from about 0.75 mg/mL to about 3 mg/mL, thereby embedding the PHH in a fibrillar collagen matrix; and
c) culturing the PHH embedded in the collagen matrix, thereby obtaining spheroids comprising proliferative PHH;
d) optionally adding an inhibitor of the MAPK MEK1/2-ERK1/2 pathway as described above to the culture of PHH embedded in the collagen matrix, thereby inducing one or more additional wave(s) of proliferation of the PHH; and
e) optionally incubating the spheroids comprising proliferative PHH embedded in the collagen matrix with a collagenase or an enzyme mixture with collagenolytic activity, thereby isolating the spheroids comprising proliferative PHH from the collagen matrix.

The present invention also relates to a method of culturing PHH, comprising:
a) first culturing the PHH in a non-adherent culture vessel as described hereinabove;
b) then transferring the PHH to a culture medium comprising methacrylated gelatin (GelMa), thereby embedding the PHH in a GelMa matrix;
c) culturing the PHH embedded in the GelMa matrix, thereby obtaining spheroids comprising proliferative PHH;
d) optionally adding an inhibitor of the MAPK MEK1/2-ERK1/2 pathway as described above to the culture of PHH embedded in the GelMa matrix, thereby inducing one or more additional wave(s) of proliferation of the PHH; and
e) optionally incubating the spheroids comprising proliferative PHH embedded in the GelMa matrix with a collagenase or an enzyme mixture with collagenolytic activity, thereby isolating the spheroids comprising proliferative PHH from the GelMa matrix.

In one embodiment, the present invention relates to a method of culturing PHH, comprising:
a) first culturing the PHH in a low or ultra-low attachment culture vessel, preferably a low or ultra-low attachment plate;
b) then transferring the PHH to a culture medium comprising methacrylated gelatin at a concentration ranging from about 1% (w/v) to about 10% (w/v), more preferably of about 5% (w/v), thereby embedding the PHH in a GelMa matrix; and
c) culturing the PHH embedded in the GelMa matrix, thereby obtaining spheroids comprising proliferative PHH;
d) optionally adding an inhibitor of the MAPK MEK1/2-ERK1/2 pathway as described above to the culture of PHH embedded in the GelMa matrix, thereby inducing one or more additional wave(s) of proliferation of the PHH; and
e) optionally incubating the spheroids comprising proliferative PHH embedded in the GelMa matrix with a collagenase or an enzyme mixture with collagenolytic activity, thereby isolating the spheroids comprising proliferative PHH from the GelMa matrix.

According to the present invention, the animal cells to be cultured with the method of the invention are primary human hepatocytes, and the method of the invention allows to obtain spheroids comprising proliferative primary human hepatocytes, wherein said spheroids have an acinus-like structure with a hollow lumen.

Thus, the invention also relates to a method for inducing *in vitro* proliferation of primary human hepatocytes, comprising:
a) first culturing the primary human hepatocytes in a non-adherent culture vessel, preferably a low or ultra-low attachment culture vessel;
b) then transferring the primary human hepatocytes to a culture medium comprising collagen or gelatin, in particular methacrylated gelatin (GelMa), as described hereinabove, thereby embedding the primary human hepatocytes in a collagen matrix or in a gelatin matrix, in particular a GelMa matrix;
c) culturing the primary human hepatocytes embedded in collagen matrix or in gelatin matrix, in particular a GelMa matrix, thereby obtaining spheroids comprising proliferative primary human hepatocytes; and
d) optionally adding an inhibitor of the MAPK MEK1/2-ERK1/2 pathway as described above to the culture of primary human hepatocytes embedded in the collagen matrix or in the gelatin matrix, in particular the GelMa matrix, thereby inducing one or more additional wave(s) of proliferation of the primary human hepatocytes.

Another object of the invention is a spheroid comprising proliferative primary human hepatocytes (PHH).

According to one embodiment of the invention, said spheroid comprises primary hepatocytes which retain their differentiated state and their hepatic functions throughout the time of their culture in the collagen matrix or in the gelatin matrix, in particular a GelMa matrix, as described hereinabove.

According to the present invention, the spheroid comprising primary human hepatocytes of the invention has an acinus-like structure with a hollow lumen.

In one embodiment, the spheroid according to the present invention thus appears as a sphere delimited by a single layer of well-organized primary human hepatocytes, forming an acinus-like structure with a hollow lumen.

Methods to assess the differentiated state of primary hepatocytes include detection and intracellular localization of epithelial markers such as E-cadherin; and detection and intracellular localization of mesenchymal markers such as N-cadherin, vimentin, cytokeratin 8, and cytokeratin 18.

As used herein, "hepatic functions" encompass the polarization, protein expression, protein secretion, and enzymatic activities specific to hepatocytes.

Thus, hepatic functions may be assessed for example though the determination of the expression of fetoprotein, albumin, and/or aldolase B; the determination of the secretion of albumin; the determination of the expression, intracellular localization and/or activity of xenobiotic metabolism enzymes such as phase I, phase II, and/or phase III enzymes; the determination of the expression, intracellular localization and/or activity of drug transporters such as MRP2 and/or MRP3.

In one embodiment, the spheroid of the invention comprises proliferative PHH, which can be further characterized as being at least one of the following:
- polarized proliferative PHH;
- proliferative PHH expressing fetoprotein, albumin, and/or aldolase B, preferably albumin, and/or aldolase B;
- proliferative PHH secreting albumin;
- proliferative PHH expressing phase I, phase II, and/or phase III metabolism enzymes; and/or
- proliferative PHH expressing drug transporters such as MRP2 and/or MRP3.

In one embodiment, the spheroid of the invention comprises polarized proliferative PHH. In one embodiment, the spheroid of the invention comprises proliferative PHH expressing fetoprotein, albumin, and/or aldolase B. In one embodiment, the spheroid of the invention comprises proliferative PHH secreting albumin. In one embodiment, the spheroid of the invention comprises proliferative PHH expressing phase I, phase II, and/or phase III metabolism enzymes. In one embodiment, the spheroid of the invention comprises proliferative PHH expressing drug transporters such as MRP2 and/or MRP3.

According to the present invention, the spheroid of the invention is embedded in a collagen matrix or in a gelatin matrix, in particular a GelMa matrix.

According to the present invention, the spheroid of the invention is obtained or susceptible to be obtained according to the method as described hereinabove. Thus, in one embodiment, the spheroid of the invention is obtained or susceptible to be obtained according to the method comprising:
a) first culturing the PHH in a low or ultra-low culture vessel, preferably in a low or ultra-low attachment plate;
b) then transferring the PHH to a culture medium comprising collagen or gelatin, in particular methacrylated gelatin (GelMa), as described hereinabove, thereby embedding the PHH in a collagen matrix or in a gelatin matrix, in particular a GelMa matrix;
c) culturing the PHH embedded in the collagen matrix or in the gelatin matrix, in particular the GelMa matrix, thereby obtaining spheroids comprising proliferative PHH.

In one embodiment, the spheroid of the invention has a diameter ranging from about 50 µm to about 150 µm.

In one embodiment, the spheroid of the invention has a diameter ranging from about 20 µm to about 130 µm, preferably from about 30 µm to about 100 µm, more preferably from about 40 µm to about 90 µm, even more preferably from about 45µm to about 80 µm.

In one embodiment, the spheroid of the invention has a volume ranging from about 4 pL to about 2000 pL, preferably about from about 100 pL to about 600 pL.

In one embodiment, the spheroid of the invention comprises from about 2 to about 150 proliferative primary human hepatocytes, preferably from about 5 to about 100 proliferative primary human hepatocytes, more preferably from about 5 to about 50 proliferative primary human hepatocytes.

The invention also relates to the use of a spheroid comprising proliferative primary human hepatocytes as described hereinabove for engineering an artificial liver model or an artificial liver organ.

The invention also relates to the use of a spheroid comprising proliferative primary human hepatocytes as described hereinabove for assessing *in vitro* the toxicity and/or the effects of a drug or a compound.

Examples of parameters that may be examined to assess the toxicity and/or the effects of a drug or a compound include cell morphology and motility, matrix reorganization and collagen synthesis, cell death, cell proliferation, cell metabolism, cell polarity, and cell differentiation.

Example of methods to assess the morphology and motility of hepatocytes include contrast light microscopy, confocal microscopy, auto-fluorescence imaging of hepatocytes or of hepatocyte spheroids by biphoton microscopy imaging; time-lapse motility imaging, and invadosomes/pMLC, MLCK/RhoKinase immunolocalizations.

Examples of methods to assess matrix reorganization and collagen synthesis include imaging of hepatocyte spheroids with Second Harmonic Generation (SHG) microscopy.

Examples of methods to assess cell death include imaging of nuclear fragmentation (TUNEL assay), methods to assess apoptosis of hepatocytes such as caspase imaging assays and caspase activity assays, and methods to assess necrosis of hepatocytes (staining with YOYO).

Examples of methods to assess proliferation of hepatocytes include biphoton cells numeration from 3D z-stack images, determination of BrdU incorporation, determination of EdU incorporation, determination of a mitotic index, and immuno-histochemical staining or measurement of the expression of Ki67, β-tubulin, cyclin D1, cyclin D2, cyclin D3, cyclin E, cyclin A2, cyclin B, Cdk1, Cdk2 and/or Aurora A.

Examples of methods to assess the xenobiotic metabolism or the hepatic functions of hepatocytes include assays to determine the enzymatic activities, protein and mRNA expression, regulation of phase I and phase II xenobiotic metabolism enzyme such as CYPs, GSTs, UGTs, NATs and associated nuclear factors CAR, PXR, AhR; MRP2 activity (related to cholestatic status) through fluorescein diacetate processing; and assays to determining lipid content such as oil red staining, or bodipy 493/503 labelling.

In one embodiment, the spheroid comprising proliferative primary human hepatocytes as described hereinabove is used for assessing the liver genotoxicity of a drug or a compound.

Examples of parameters that may be examined to assess the liver genotoxicity of a drug or a compound include hepatocyte proliferation, DNA replication, chromosomes aberrations (such as, for example, single and double-strand breaks, loss, formation of micronuclei), presence of DNA damages, and presence of mutations.

Thus, for example, the liver genotoxicity of a drug or a compound may be assessed through detection of micronuclei formation, comet assay, detection of phosphorylation of the histone H2Ax, and exome sequencing (for example to establish hotspot signatures linked to xenobiotic exposure) performed on spheroids comprising proliferative PHH according to the present invention.

The invention also relates to an *in vitro* method of assessing the toxicity and/or the effects of a drug or a compound, said *in vitro* method comprising:
a) obtaining spheroids comprising proliferative primary human hepatocytes (PHH) according to the method as described hereinabove;
b) contacting the spheroids comprising proliferative PHH with a drug or a compound; and
c) assessing the toxicity and/or the effects of the drug or the compound on the spheroids comprising proliferative PHH.

As mentioned hereinabove, the parameters that may be examined to assess the toxicity and/or the effects of a drug or a compound on the spheroids comprising proliferative PHH include cell morphology and motility, matrix reorganization and collagen synthesis, cell death, cell proliferation, cell metabolism, cell polarity, and cell differentiation.

In one embodiment, the method of the invention is for assessing the liver genotoxicity of a drug or a compound.

As mentioned hereinabove, the parameters that may be examined to assess the liver genotoxicity of a drug or a compound on the spheroids comprising proliferative PHH, include hepatocyte proliferation, DNA replication, presence of chromosomes aberrations (such as, for example, single-strand breaks or double-strand breaks, loss, micronuclei formation), presence of DNA damages, and presence of mutations.

Thus, the liver genotoxicity of a drug or a compound may be assessed, for example, through detection of micronuclei, comet assay, detection of the presence of phosphorylation of the histone H2Ax, and exome sequencing (for example to establish xenobiotic signatures) performed on spheroids comprising proliferative PHH according to the present invention.

In one embodiment, the spheroids comprising proliferative PHH are embedded in the collagen matrix or in the gelatin matrix, in particular the GelMa matrix, as described hereinabove.

In one embodiment, the spheroids comprising proliferative PHH are contacted with a drug or a compound after at least 2, 3, 4, 5, 6, 7, 8, 9, 10, 11, 12, 13, 14, or 15 days of culture in the collagen matrix or in the gelatin matrix, in particular the GelMa matrix. In another embodiment, the spheroids comprising proliferative PHH are contacted with a drug or a compound after at most 15, 14, 13, 12, 11, 10, 9, 8, 7, 6, 5, 4, 3, or 2 days of culture in the collagen matrix or in the gelatin matrix, in particular the GelMa matrix.

The method of the invention for culturing PHH allows to obtain spheroids which comprise proliferative PHH, said proliferative PHH retaining their phenotype, *e.g.,* their differentiated state, and their functions.

As illustrated in the Examples below, the Applicant showed that the method of the invention is particularly suited for the culture of primary human hepatocytes and allows to obtain spheroids comprising proliferative primary human hepatocytes. Of particular interest, the method of the invention allows to obtain spheroids comprising proliferative primary human hepatocytes even in the absence of growth factors such as HGF (hepatocyte growth factor) and/or EGF (epidermal growth factor). The method of the invention also allows to obtain spheroids comprising proliferative primary human hepatocytes even in the absence of FCS (fetal calf serum).

In particular, the Applicant surprisingly found that primary human hepatocytes retain their differentiated state and their hepatic functions and are able to undergo a first wave of proliferation comprising at least two cell cycles during the first week of culture in the collagen matrix or in a gelatin matrix, in particular a GelMa matrix, according to the method of the invention. The Applicant also found that primary human hepatocytes are able to undergo a second wave of proliferation during the second week of culture in the collagen matrix according to the method of the invention. Further additional wave(s) of proliferation can occur when the MEK/ERK is transiently inhibited with an inhibitor such as the MEK inhibitor U0126.

Notably, the Applicant showed that proliferation of the primary human hepatocytes only occurs when the primary human hepatocytes are first cultured in a non-adherent culture vessel such as a low attachment culture vessel, before being embedded and cultured in a collagen matrix or in a gelatin matrix, in particular a GelMa matrix, according to the method of the invention as described hereinabove. Primary human hepatocytes continuously cultured in a low attachment culture vessel, without ever being embedded in a collagen matrix or in a gelatin matrix, in particular a GelMa matrix, for a similar time and in the same culture medium, were not able to proliferate. Similarly, primary human hepatocytes directly embedded and cultured in a collagen matrix, for a similar time and in the same culture medium, were not able to proliferate.

The method of the invention thus allows to obtain non-transformed proliferative human hepatocytes essential for the further development of *in vitro* models with both therapeutic and pharmaceutical applications. For example, the capacity to expand primary human hepatocytes will most likely have an impact on the development of hepatocyte, spheroid or organoid transplant aiming at restoring liver function in a subject. The capacity to expand primary human hepatocytes will also improve *in vitro* PHH models used to predict the liver toxicity, and in particular the liver genotoxicity, of candidate drugs and environmental contaminants.

### BRIEF DESCRIPTION OF THE DRAWINGS

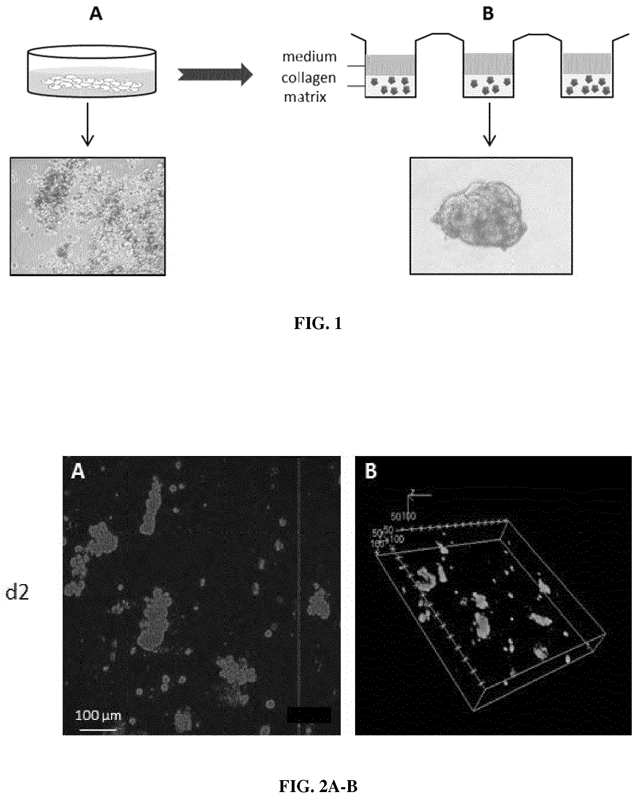

**Figure 1** is a scheme illustrating the method of the invention for culturing primary human hepatocytes. (**A**) Illustration of the first step of the method of the invention: PHH are cultured in a non-adherent culture vessel, such as a low or ultra-low attachment plate. (**B**) Illustration of the second and third steps of the method of the invention: the PHH first cultured in a non-adherent culture vessel are embedded in a collagen matrix or in a gelatin matrix, in particular a GelMa matrix (second step) and cultured within said matrix (third step).

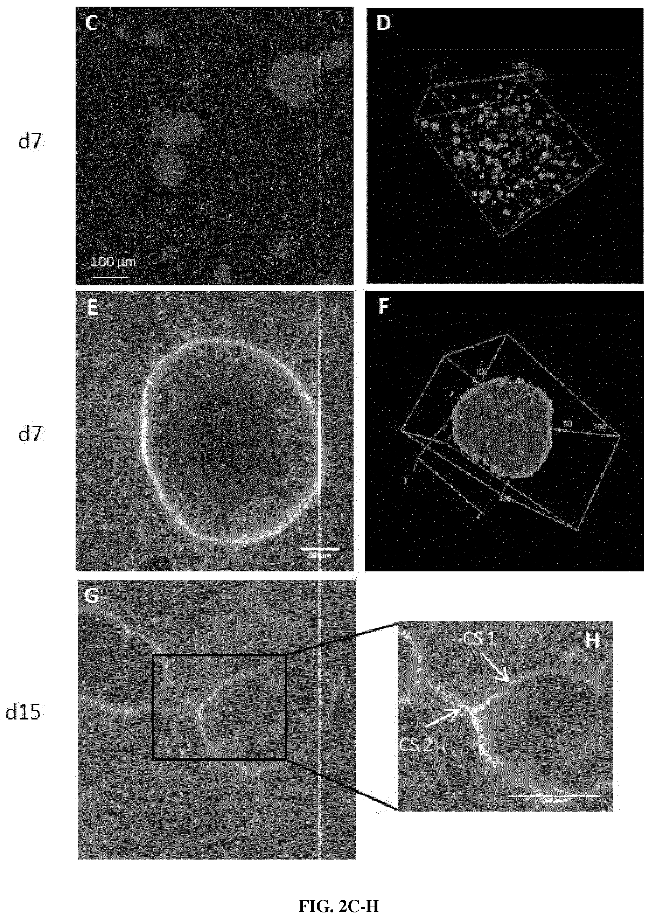

**Figure 2** is a group of photographs showing the formation of spheroids of primary human hepatocytes (PHH) in 3D cultures in a collagen matrix. The photographs were taken using two-photon excited fluorescence (TPEF) microscopy, after 2 days (**A, B**) or after 7 days (**C, D, E, F**) of 3D cultures established according to the method of the invention. 100µm depth image stack (TPEF stack) were used for 3D reconstruction (**B, D, F**). Second Harmonic Generation (SHG) microscopy was used after 15 days of 3D cultures established according to the method of the invention in order to analyze collagen signatures (CS) around (CS1) and between (CS2) PHH spheroids (**G, H**). **A, C** Bar scale = 100 µm; **E** Bar scale = 20 µm, **H** Bar scale = 50 µm.

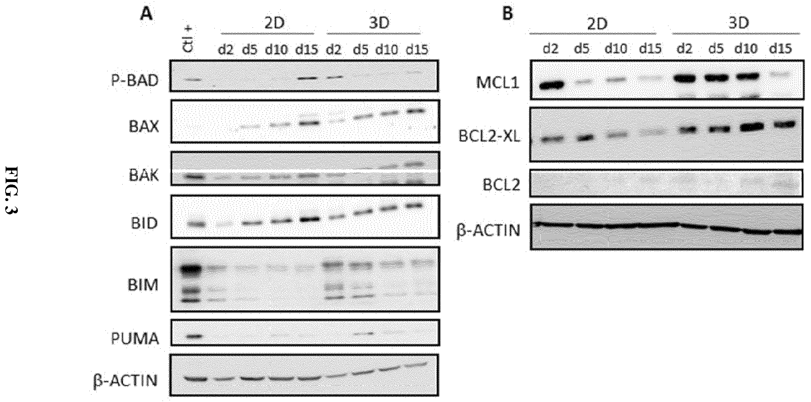

**Figure 3** is group of western-blots showing the expression in PHH of (**A**) pro-apoptotic factors (phospho-BAD, BAX, BAK, BID, BIM and PUMA) and (**B**) pro-survival factors (MCL1, BCL2-XL, BCL2) after 2, 5, 10 and 15 days of culture in control 2D cultures (2D) or in 3D cultures established according to the method of the invention in a collagen matrix (3D). β-actin is used as a loading control. Data are representative of at least three independent experiments.

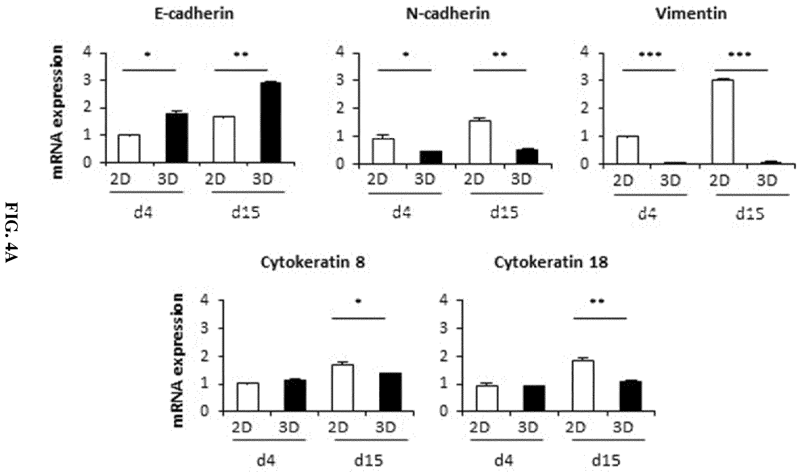

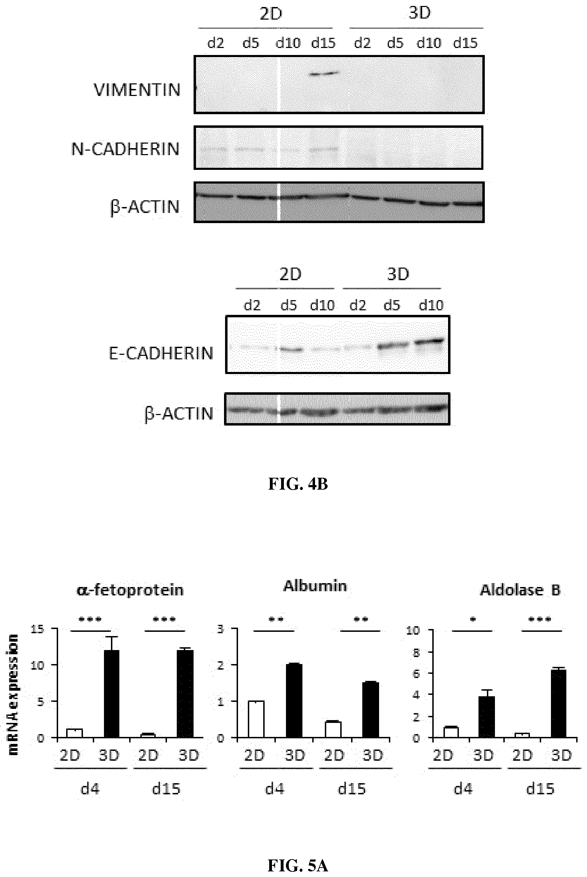

**Figure 4** is a group of histograms (**A**) and western-blots (**B**) showing the expression of factors implicated in the mesenchymal-to-epithelial transition (MET). (**A**) Histograms showing the mRNA expression of E-cadherin, N-cadherin, vimentin, cytokeratin 8 and cytokeratin 18 in PHH after 4 and 15 days of culture in control 2D cultures (white) or in 3D cultures established according to the method of the invention in a collagen matrix (black). The mRNA expressions correspond to a ratio with respect to the expression level after 4 days of culture in control 2D cultures. (**B**) Western-blots showing the expression of vimentin, N-cadherin, and E-cadherin in PHH after 2, 5, 10 and 15 days of culture in control 2D cultures (2D) or in 3D cultures established according to the method of the invention in a collagen matrix (3D). β-actin is used as a loading control.

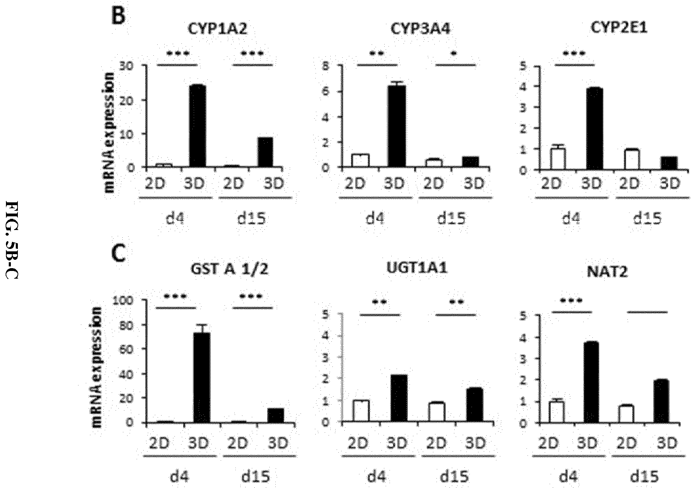

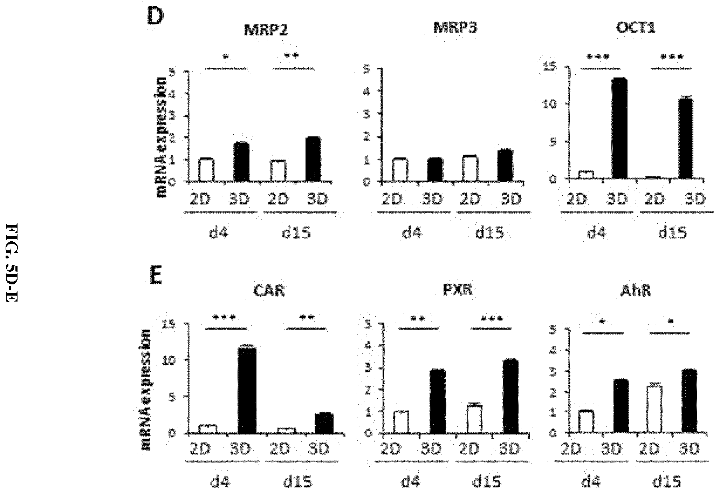

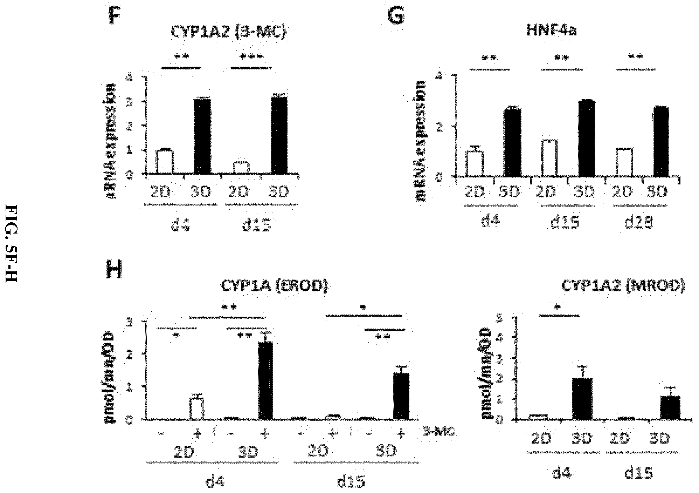

**Figure 5** is a group of histograms showing the mRNA expression of: hepatocyte differentiation markers (**A**), drug-metabolizing enzymes of phase I (**B**), drug-metabolizing enzymes of phase II (**C**), drug transporters (**D**), CYP regulators (**E**), CYP1A2 after 3-MC induction for 24h (5µM) (**F**) and HNF4α (**G**) in PHH after 4, 15 and 28 days of culture (the latter for HNF4α only) in control 2D cultures (white) or in 3D cultures established according to the method of the invention in a collagen matrix (black). The mRNA expressions correspond to a ratio with respect to the expression level after 4 days of culture in control 2D cultures. The indicated values were obtained from one experiment representative of at least three independent experiments (mean ± SD, * p<0.05, ** p<0.01, *** p<0.001). (**H**) Histograms showing CYP1A (EROD) activity after 3-MC induction for 24h (5 µM) and basal CYP1A2 (MROD) activity in PHH after 4 and 15 days of culture in control 2D cultures (white) or in 3D cultures established according to the method of the invention in a collagen matrix (black).

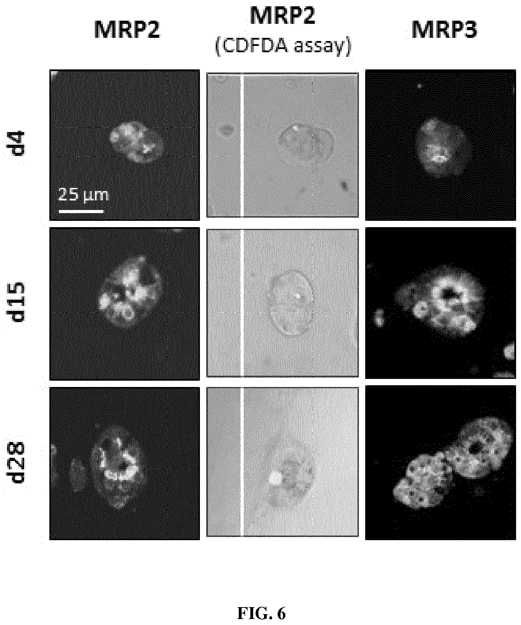

**Figure 6** is a group of photographs obtained with fluorescence microscopy showing the localization of the drug transporters MRP2 and MRP3, of albumin and of nuclei in PHH spheroids after 4, 15 and 28 days in 3D cultures established according to the method of the invention in a collagen matrix. MRP2 functional activity is evaluated using the fluorescein diacetate CDFDA assay showing a clear efflux of the substrate in the central lumen of the PHH spheroids after 4, 15 and 28 days in 3D cultures established according to the method of the invention in a collagen matrix. Bar scale = 25 µm.

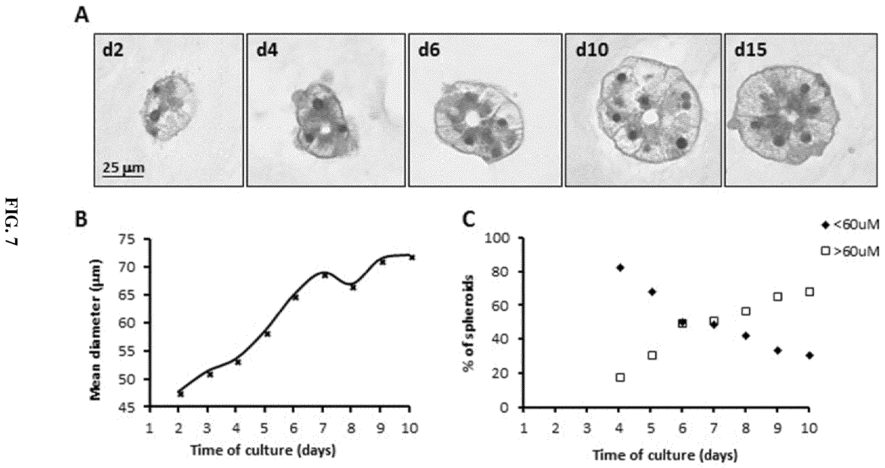

**Figure 7A** is a group of photographs obtained after HES staining showing PHH spheroids after 2, 4, 6, 10 and 15 days in 3D cultures established according to the method of the invention in a collagen matrix. Bar scale = 25 µm. **Figures 7B-C** are a group of graphs showing the evolution of the mean diameter (µm) of PHH spheroids as a function of culture time (**B**) and the number of PHH spheroids with a diameter < 60 µm and the number of PHH spheroids with a diameter > 60 µm as a function of culture time (**C**).

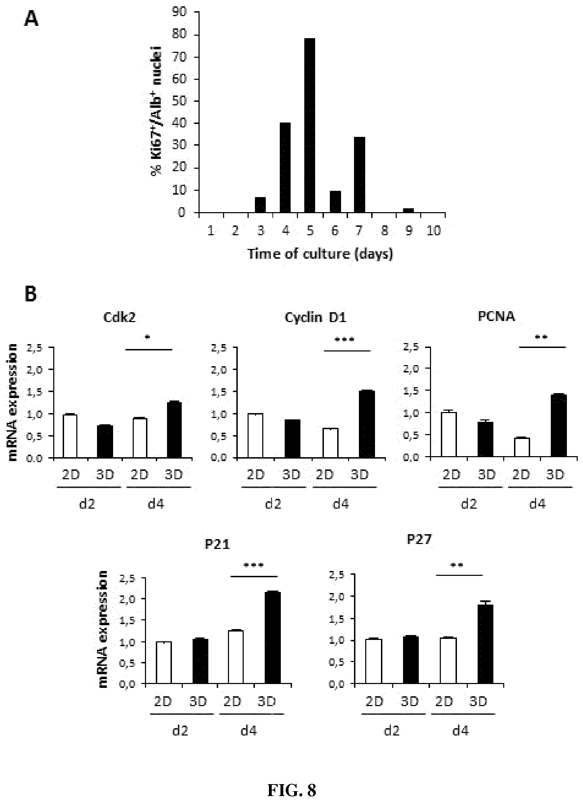

**Figure 8** is a group of graphs showing: (**A**) the proliferation over time of PHH in a 3D culture established according to the method of the invention in a collagen matrix (expressed as the percentage of nuclei that are both Ki67⁺ and Alb⁺), based on the quantification of the expression of the proliferation marker Ki67 and albumin in nuclei; (**B**) the mRNA expression of the proliferation markers Cdk2, CyclinD1, PCNA, P21, P27 in PHH after 2 and 4 days of culture in control 2D cultures (white) or in 3D cultures established according to the method of the invention in a collagen matrix (black). The mRNA expressions correspond to a ratio with respect to the expression level at 2 days of culture in control 2D cultures.

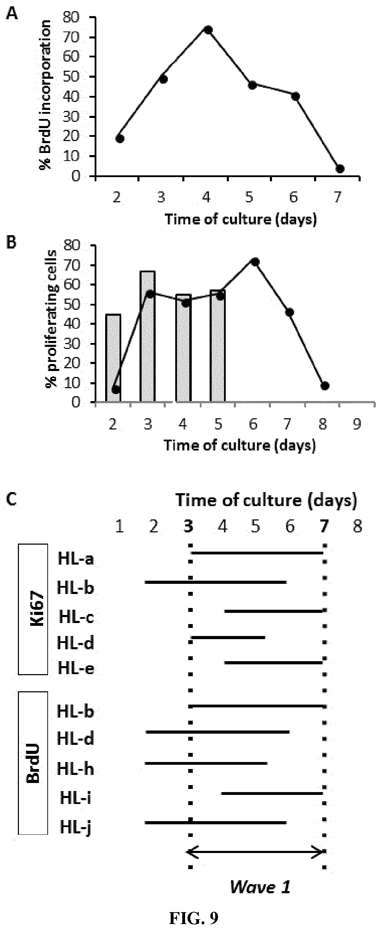

**Figure 9** is a group of graphs showing: (**A**) the quantification of the incorporation of BrdU in a 3D culture established according to the method of the invention in a collagen matrix; (**B**) the proliferation over time of PHH in a 3D culture established according to the method of the invention in a collagen matrix (expressed as the percentage of proliferating cells), based on the simultaneous quantification of the proliferation marker Ki67 (grey boxes) and of the incorporation of BrdU (black line); (**C**) the representation of the wave of proliferation between days 2 and 7 in eight 3D cultures established according to the method of the invention in a collagen matrix from hepatocytes of different donors as detected through Ki67 staining and BrdU incorporation as indicated.

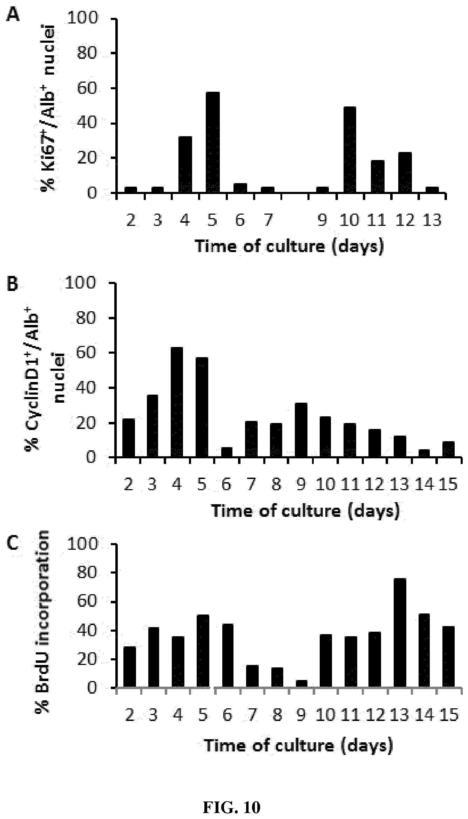

**Figure 10** is a group of histograms showing the existence of two waves of proliferation in PHH in 3D culture established according to the method of the invention in a collagen matrix through the (**A**) quantification of the percentage of Ki67⁺/Alb⁺nuclei over 13 days of 3D culture established according to the method of the invention in a collagen matrix; (**B**) quantification of the percentage of Cyclin D1⁺/Alb⁺ nuclei over 15 days of 3D culture established according to the method of the invention in a collagen matrix; (**C**) quantification of the BrdU incorporation over 15 days of 3D culture established according to the method of the invention in a collagen matrix.

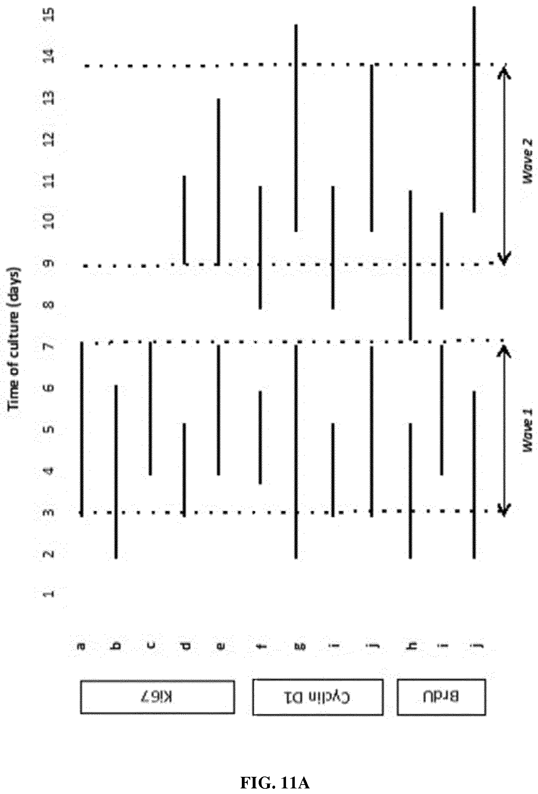

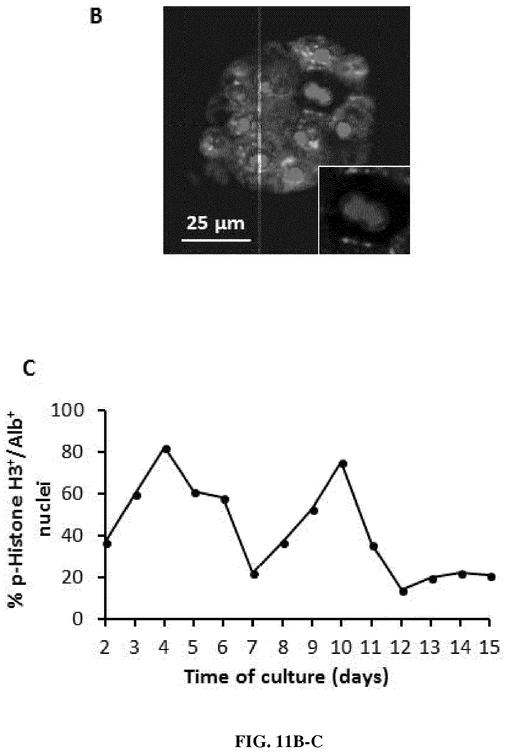

**Figure 11A** is a scheme illustrating the waves of proliferation occurring in the 3D culture established according to the method of the invention in a collagen matrix from hepatocytes of different donors during the first week of culture (first wave of proliferation: on average between day 3 and day 7) and during the second week of culture (second wave of proliferation: on average between day 9 and day 14). **Figure 11B** is an illustration of an immunostaining of phospho-histone H3, a marker of cells in the M phase of the cell cycle, of albumin and of nuclei in a PHH spheroid after 10 days in 3D culture established according to the method of the invention in a collagen matrix. Bar scale = 25 µm. **Figure 11C** is a quantification of phospho-histone H3⁺/Alb⁺ nuclei over 15 days of 3D culture established according to the method of the invention in a collagen matrix.

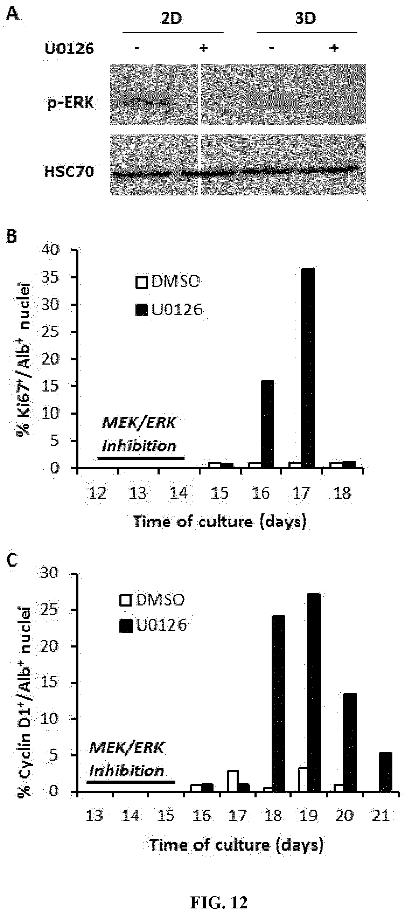

**Figure 12** illustrates the proliferation observed in PHH after transient blockage of the MEK-ERK pathway. (**A**) Western-blot showing the inhibition of ERK phosphorylation after 14 days of culture in control 2D culture (2D) or in 3D culture established according to the method of the invention in a collagen matrix (3D) with addition of U0126 (+) or DMSO (-) between day 12 and day 14. (**B**) Proliferation between day 12 and day 18 of PHH in 3D culture established according to the method of the invention in a collagen matrix after MEK inhibition with U0126 between day 12 and day 14. Proliferation is assessed through the quantification of the proliferation marker Ki67 (expressed as the percentage of nuclei that are both Ki67⁺ and Alb⁺). A negative control was carried out using DMSO instead of U0126. (**C**) Proliferation between day 13 and day 21 of PHH in 3D culture established according to the method of the invention in a collagen matrix after MEK inhibition with U0126 between day 15 and day 17. Proliferation is assessed through the quantification of the proliferation marker cyclin D1 (expressed as the percentage of nuclei that are both cyclinD1⁺ and Alb⁺). A negative control was carried out using DMSO instead of U0126.

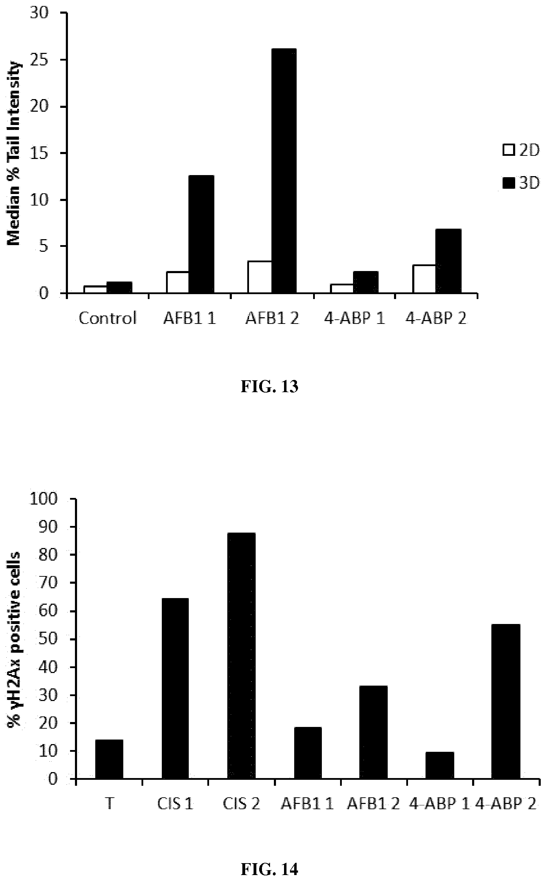

**Figure 13** is a histogram showing the DNA damage evaluated by the percentage of tail DNA in individual PHH after 9 days of incubation with the indicated genotoxic drugs at the indicated concentrations either in control 2D culture (2D) or in 3D culture established according to the method of the invention in a collagen matrix (3D). Control: no genotoxic drug; AFB1 1: 0.1 µM; AFB1 2: 0.5 µM; 4-ABP 1: 1 µM; 4-ABP 2: 10 µM.
**Figure 14** is a histogram showing the quantification of the number of γH2Ax-positive PHH assessed by immunostaining after 9 days of incubation with the indicated genotoxic drugs at the indicated concentrations in 3D culture established according to the method of the invention in a collagen matrix (3D). T: no genotoxic drug; CIS 1: 5 µg/mL cisplatin; CIS 2: 10 µg/mL cisplatin; AFB1 1: 0.1 µM AFB1; AFB1 2: 0.5 µM; 4-ABP 1: 1 µM; 4-ABP 2: 10 µM.

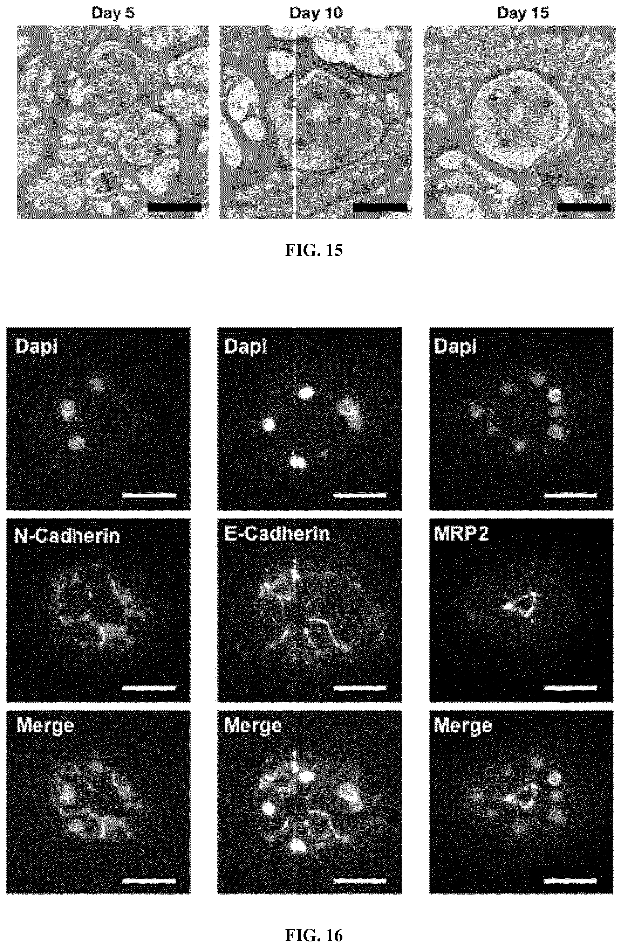

**Figure 15** is a group of photographs is a group of photographs obtained after HES staining showing PHH spheroids after 5, 10 and 15 days in 3D cultures established according to the method of the invention in a methacrylated gelatin (GelMa) matrix. Bar scale = 50 µm. Data are representative of three independent experiments.
**Figure 16** is a group of photographs obtained with fluorescence microscopy showing the localization of N cadherin, of albumin and of nuclei in PHH spheroids after 10 days in 3D cultures established according to the method of the invention in a methacrylated gelatin (GelMa) matrix. Bar scale = 50 µm. Data are representative of three independent experiments.

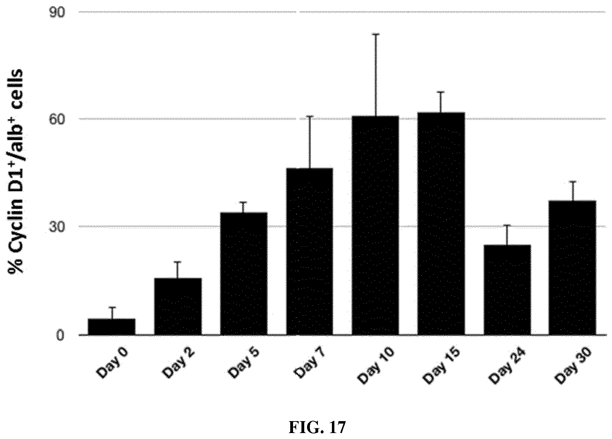

**Figure 17** is a histogram showing the proliferation over time of PHH in a 3D culture established according to the method of the invention in a methacrylated gelatin (GelMa) matrix (expressed as the percentage of cells that are both cyclin D1⁺ and Alb⁺), based on the quantification of the expression of the proliferation marker cyclin D1 and albumin in PHH.

### EXAMPLES

The present invention is further illustrated by the following examples.

### Example 1:

### Materials and Methods

### Material

### Cells

Human liver samples were obtained from patients undergoing liver resection for primary hepatocellular carcinoma or liver metastases through the Centre de Ressources Biologiques (CRB)-Santé of Rennes (CHRU Pontchaillou, Rennes, France). The research protocol was conducted under French legal guidelines and the local institutional ethics committee. The clinical characteristics of the human liver samples are detailed in **Table 1** below.

**Table 1: clinical characteristics of the donors from whom the human liver samples were obtained**

| Case | Age | Gender | Liver pathology | Smoker | Diabetic |
|---|---|---|---|---|---|
| HL-a | 68 | F | Cholangiocarcinoma | yes | no |
| HL-b | 68 | M | Cholangiocarcinoma | no | no |
| HL-c | 66 | F | Liver metastasis from colorectal carcinoma | no | no |
| HL-d | 72 | F | Liver metastasis from colorectal carcinoma | no | no |
| HL-e | 56 | M | Cholangiocarcinoma | no | no |
| HL-f | 59 | M | Liver metastasis from colorectal carcinoma | no | no |
| HL-g | 60 | F | Liver metastasis from colorectal carcinoma | no | no |
| HL-h | 78 | M | Liver metastasis from stromal tumor | no | yes |
| HL-i | 75 | M | Hepatocellular Carcinoma | no | yes |
| HL-j | 60 | M | Liver metastasis from colorectal carcinoma | no | no |

Human hepatocytes were isolated by a two-step collagenase perfusion procedure, and parenchymal cells were maintained in a modified William's E medium, called WE HH (human hepatocytes) comprising penicillin (100 U), streptomycin (100 µg/mL), insulin (15 µg/mL), glutamine (2 mM), albumin (0.1% (w/v)), transferrin (5.5 µg/mL), sodium selenite (5 µg/mL), hydrocortisone (1 µM), HGF (hepatocyte growth factor) (2.5 ng/mL), EGF (epidermal growth factor) (0.05 ng/µL) with or without FCS (fetal calf serum) (10% (v/v)).

### Reagents

The cell proliferation reagent WST-1, Liberase (10 µg/mL) and colcemid (1 µM) were obtained from Roche. Cisplatin was obtained from Mylan. Type I collagen, ethoxyresorufin, methoxyresorufin, Hoechst, 5(6)-Carboxy-2',7'-dichlorofluorescein diacetate (CDFDA, 10 µM), 1X ITS (insulin, transferrin, selenium) and anti β-actin antibody (A-5441, 1/1000) were obtained from Sigma-Aldrich. BrdU (RPN201V, 1:1000) and the anti-BrdU antibody (RPN202, 1:100) were obtained from GE Healthcare (Chicago, USA). The rhEGF was obtained from Promega (Fitchburg, USA) and the rhHGF from R&D Systems. The anti-HSC-70 antibody (sc-7298, 1:5000) was obtained from Santa-Cruz Biotechnology. The anti-Ki67 (MA5-14520, 1:400) antibody was obtained from Invitrogen. Antibodies against phospho-p44/42 MAPK (Thr202/Tyr204) (9106, 1:1000), E-cadherin (3195, 1:100), pro-apoptosis Bcl-2 family (1:500), and pro-survival Bcl-2 family (1:500) were obtained from Cell Signaling. The anti-N-cadherin antibody (610921, 1:100) was obtained from BD Biosciences. Antibodies against MRP2 (ab3373, 1:100) and Cyclin D1 (ab16663, 1:100) were obtained from Abcam. The anti-vimentin antibody (M0725, 1:1000) was from obtained Dako. The anti phospho-histone H3 (Ser10) antibody (06-570, 1:100) was obtained from Merck and the anti-albumin antibody (A80-229A, 1:100) was obtained from Bethyl Laboratories, Inc. The MEK inhibitor U0126 (50 µM) was obtained from Promega.

### Methods

### Primary human hepatocyte 2D culture

As a control, aggregates of primary human hepatocytes (PHH) formed in low attachment plates as described below were seeded in standard multi-well plates and cultured in the same medium, *i.e.,* the WE HH medium described hereinabove.

### Primary human hepatocyte 3D culture

As shown on **Figure 1****,** primary human hepatocytes (PHH) isolated as indicated above were first cultured in a low attachment plate **(****Figure 1A****).** Then, the aggregates of PHH thus obtained were embedded in a collagen matrix (also referred to as collagen gel) and further cultured in the collagen matrix **(****Figure 1B****).**

*Culture in a low attachment plate*: isolated human hepatocytes were incubated at 37°C, 5% CO2, humidity 85-95% during one night (about 15 to 20h) in 6-well low attachment plates (Corning^{®}, Costar^{®}) at a concentration of 2x10⁶ to 2.5x10⁶ cells per well, in WE or WE HH.

*Inclusion in a collagen matrix followed by culture of hepatocytes embedded in the collagen matrix*: type I collagen at 3 mg/mL or 6 mg/mL from Sigma-Aldrich was diluted into WE HH culture medium to obtain a collagen solution at the desired concentration. The human hepatocytes were added at a concentration of 3.65x10⁵ cells/mL to the WE HH comprising collagen and the pH was adjusted at 7,4 with NaOH 0.1 N. The resulting mix of human hepatocytes and DMEM HH comprising collagen was poured into 96-well plates (100µl) or 48-well plates (300µl) and incubated at 37°C, 5% CO2, humidity 85-95%. After at least 2h, the gels were polymerized and a volume of WE HH medium equal to the volume of gel was added. The hepatocytes embedded in the gel matrix were cultured in the modified WE HH as described above for at least 2 days at 37°C, 5% CO2, humidity 85-95%. The inclusion of the hepatocytes in a collagen matrix thus allowed for a 3D culture of the hepatocytes.

Hereafter, in Example 1, the term "3D culture" refers to a culture of PHH established as described hereinabove, with a first incubation (or culture) of the PHH in a low attachment plate followed by the inclusion of the PHH in a collagen matrix, according to the method of the invention.

However, any mention of a time of culture, *e.g.*, day 5 of culture, refers to the time of culture in a collagen matrix (or in a standard multi-well plate for the 2D control condition). In other words, any time of culture mentioned hereafter does not include the period of incubation (or culture) in a low attachment plate.

### Collagen gels inclusion in paraffin

After fixation in formol (also known as formaldehyde) 4%, collagens gels were dehydrated by successive incubations in alcohol and xylene baths at increasing concentrations before being impregnated with paraffin using EXCELSIOR ES tools (Thermo Fisher Scientific, Waltham, USA). After impregnation, gels were included in paraffin blocs and 4 µm cuts were made.

### Hepatocyte DNA synthesis

Incorporation of the thymidine analog BrdU was used as an index of cell proliferation. After 24h of incorporation, cells in collagen gels were fixed in ethanol-glycine and included in paraffin as indicated above. BrdU positive cells were detected using an anti-BrdU antibody (RPN202, 1:100).

### Immunohistochemistry

Immuno-histochemical staining was performed on the Discovery Automated IHC stainer using the Discovery Rhodamine kit (Ventana Medical Systems, Tucson, USA). Following deparaffination with Ventana EZ Prep solution at 75°C for 8 min, antigen retrieval was performed using Tris-based buffer solution CC1 (Ventana Medical Systems, Tucson, USA) at 95°C to 100°C for 36 min. Endogen peroxidase was blocked with 3% H₂O₂ (Ventana Medical Systems, Tucson, USA) for 8 min at 37°C. After rinsing with reaction buffer (Roche, Basel, Switzerland), slides were incubated at 37°C for 60 min with an appropriate dilution of the desired primary antibodies. After rinsing, signal enhancement was performed using the Ventana Rhodamine kit and incubation with the secondary antibody anti-Rabbit HRP (Roche, Basel, Switzerland) was carried out for 16 min. After removal from the instrument, slides were manually rinsed, stained with albumin (1:100), with the secondary antibody for albumin detection (Donkey anti-goat 655, 1:250) and with Hoechst (1:1500), and coverslipped.

### RT-qPCR analysis

Cells were extracted from the collagen gels by the action of the Liberase enzyme blend 10 µg/mL (Roche, Basel, Switzerland) for 15 min at 37°C. Then, total RNA was extracted from cell pellets using NucleoSpin RNA (Macherey-Nagel, Hoerdt, France) and the concentration of total RNA was measured with a NanoDrop ND-1000 (NanoDrop Technologies, Wilmington, USA). Total RNA was used for cDNA synthesis using the High Capacity cDNA reverse transcription kit (Applied Biosystems, Saint Aubin, France). Real-time PCR for all genes was performed using SYBR green technology with the Power SYBR Green PCR master mix (Applied Biosystems, Saint Aubin, France) and the CFX384 Real-Time System (Biorad) according to the manufacturer's recommendations. Primer sequences used are described in **Tables 2** and **3** below.

The amplification curves were analyzed with the Biorad CFX Manager software using the comparative regression method. GAPDH was used for the normalization of expression data. The relative amount of measured mRNA in samples was determined using the 2-ΔΔCT method where ΔΔCT = (CTtarget - CTGAPDH)ₛₐₘₚₗₑ - (CTtarget-CTGAPDH)_{calibrator}. Final results were expressed as the n-fold differences in target gene expression in tested samples when compared with the mean expression value of calibrator. Values are given for one experiment representative of at least three independent experiments.

**Table 2: forward primer sequences**

| Gene | Forward primer | SEQ ID NO |
|---|---|---|
| GAPDH | GTGGACCTGACCTGCCGTCT | SEQ ID NO: 1 |
| E-CADHERIN | TGCTCTTGCTGTTTCTTCGG | SEQ ID NO: 2 |
| N-CADHERIN | GTGCATGAAGGACAGCCTCT | SEQ ID NO: 3 |
| VIMENTIN | CCAAACTTTTCCTCCCTGAACC | SEQ ID NO: 4 |
| CYTOKERATIN 8 | AGCTGGAGTCTCGCCTGGAA | SEQ ID NO: 5 |
| CYTOKERATIN 18 | GAGCCTGGAGACCGAGAAC | SEQ ID NO: 6 |
| α-FETOPROTEIN | TGCAGCCAAAGTGAAGAGGGAAGA | SEQ ID NO: 7 |
| ALBUMIN | GGGCATGTTTTTGTATGAAT | SEQ ID NO: 8 |
| ALDOLASE B | AGAATGGACTGGTACCTATTG | SEQ ID NO: 9 |
| CYP1A2 | TGGAGACCTTCCGACACTCCT | SEQ ID NO: 10 |
| CYP3A4 | CTTCATCCAATGGACTGCATAAAT | SEQ ID NO: 11 |
| CYP2E1 | CCTGCTCGTGGAAATGGAGA | SEQ ID NO: 12 |
| GSTA1/2 | CGCTACTTCCCTGCCTTTGA | SEQ ID NO: 13 |
| UGT1A1 | TGACGCCTCGTTGTACATCAG | SEQ ID NO: 14 |
| NAT2 | AGGAACAAATTGGACTTGGA | SEQ ID NO: 15 |
| MRP2 | TGAGCAAGTTTGAAACGCACAT | SEQ ID NO: 16 |
| MRP3 | GTCCGCAGAATGGACTTGAT | SEQ ID NO: 17 |
| OCT1 | TAATGGACCACATCGCTCAA | SEQ ID NO: 18 |
| CAR | TGATCAGCTGCAAGAGGAGA | SEQ ID NO: 19 |
| PXR | CCAGGACATACACCCCTTTG | SEQ ID NO: 20 |
| AhR | CTTCCAAGCGGCATAGAGAC | SEQ ID NO: 21 |
| CDK2 | CATTCCTCTTCCCCTCATCA | SEQ ID NO: 22 |
| CYCLIN D1 | CCGTCCATGCGGAAGATC | SEQ ID NO: 23 |
| PCNA | ATCATTACACTAAGGGCCGAAGATAAC | SEQ ID NO: 24 |
| P21 | TGAGCCGCGACTGTGATG | SEQ ID NO: 25 |
| P27 | CATTTGGTGGACCCAAAGAC | SEQ ID NO: 26 |
| HNF4a | CAGAAGGCACCAACCTCAAC | SEQ ID NO: 27 |

**Table 3: reverse primer sequences**

| Gene | Reverse primer | SEQ ID NO |
|---|---|---|
| GAPDH | GGAGGAGTGGGTGTCGCTGT | SEQ ID NO: 28 |
| E-CADHERIN | TGCCCCATTCGTTCAAGTAG | SEQ ID NO: 29 |
| N-CADHERIN | ATGCCATCTTCATCCACCTT | SEQ ID NO: 30 |
| VIMENTIN | GTGATGCTGAGAAGTTTCGTTGA | SEQ ID NO: 31 |
| CYTOKERATIN 8 | TGTGCCTTGACCTCAGCAATG | SEQ ID NO: 32 |
| CYTOKERATIN 18 | TTGCGAAGATCTGAGCCC | SEQ ID NO: 33 |
| α-FETOPROTEIN | CATAGCGAGCAGCCCAAAGAAGAA | SEQ ID NO: 34 |
| ALBUMIN | CCCACTTTTCCTAGGTTTCT | SEQ ID NO: 35 |
| ALDOLASE B | GTAACATACTGGCAGTGTTC | SEQ ID NO: 36 |
| CYP1A2 | CGTTGTGTCCCTTGTTGTGC | SEQ ID NO: 37 |
| CYP3A4 | TCCCAAGTATAACACTCTACACAGACAA | SEQ ID NO: 38 |
| CYP2E1 | TCTCTGTCCCCGCAAAGAAC | SEQ ID NO: 39 |
| GSTA1/2 | AGTCAAGCTCCTCGACGTAG | SEQ ID NO: 40 |
| UGT1A1 | CCTCCCTTTGGAATGGCAC | SEQ ID NO: 41 |
| NAT2 | ACCCCGGTTTCTTCTTACAA | SEQ ID NO: 42 |
| MRP2 | AGCTCTTCTCCTGCCGTCTCT | SEQ ID NO: 43 |
| MRP3 | TCACCACTTGGGGATCATTT | SEQ ID NO: 44 |
| OCT1 | AGCCCCTGATAGAGCACAGA | SEQ ID NO: 45 |
| CAR | AGGCCTAGCAACTTCGCATA | SEQ ID NO: 46 |
| PXR | CTACCTGTGATGCCGAACAA | SEQ ID NO: 47 |
| AhR | AG TTATCCTGGCCTCCGTTT | SEQ ID NO: 48 |
| CDK2 | TTTAAGGTCTCGGTGGAGGA | SEQ ID NO: 49 |
| CYCLIN D1 | GAAGACCTCCTCCTCGCACT | SEQ ID NO: 50 |
| PCNA | TCATTTCATAGTCTGAAACTTTCTCCTG | SEQ ID NO: 51 |
| P21 | GTCTCGGTGACAAAGTCGAAGTT | SEQ ID NO: 52 |
| P27 | AGAAGAATCGTCGGTTGCAG | SEQ ID NO: 53 |
| HNF4a | CTCGAGGCACCGTAGTGTTT | SEQ ID NO: 54 |

### Immunoblotting analysis

Cells were extracted from the collagen gels by the action of the Liberase enzyme blend 10 µg/mL (Roche, Basel, Switzerland) for 15 min at 37°C. Protein samples were extracted from cell pellets and dosed. Protein samples were then separated using SDS-PAGE and transferred onto nitrocellulose membranes in a transfer buffer (25 mM Tris, 200 mM glycine, Ethanol 20%). The blots were blocked with 5% low fat milk in Tris-buffer saline (TBS) (65 mM Tris pH 7.4, 150 mM NaCl) at room temperature for 1h. The blots were incubated overnight with the desired primary antibodies at 4°C. The blots were washed with TBS and incubated for 1h with a mouse-IgG HRP or a rabbit-IgG HRP secondary antibody (1:1000) in 5% low fat milk in TBS at room temperature. The blots were then washed with TBS. Immunocomplexes were visualized with a Fujifilm LAS-3000 imager (Fujifilm, Tokyo, Japan) after a chemiluminescent reaction using the Immobilon Western Chemiluminescent HRP substrate (Millipore, Merck, Darmstadt, Germany). Densitometric analyses of the bands were carried out with MultiGauge software (Fujifilm, Tokyo, Japan).

### MRP2 transporter activity

A fluorescence-based efflux assay was used to investigate MRP2 transporter activity in 3D primary human hepatocyte (PHH) cultures. The membrane permeable and non-fluorescent substrate 5-(and-6)-carboxy-2',7'-dichlorofluorescein diacetate (CDFDA) enters into hepatocytes where its hydrolysis by intracellular non-specific esterases results in a fluorescent product. This product, which is a substrate of the membrane transporter MRP-2, is effluxed from hepatocytes into the bile canaliculi.

The 3D cultures were incubated 10 min with CDFDA (10 µM). The dye-solution was then replaced by serum-free medium for 2h. The cultures were washed twice with PBS solution before the fluorescence was assessed using microscopy.

### CYP activities measurement

Ethoxyresorufin O-deethylation (EROD) and methoxyresorufin O-deethylation (MROD) associated with CYP 1A1/2 and 1A2 activities, respectively, were measured in cultured hepatocytes as described by Burke and Mayer (Burke and Mayer, 1983). Briefly, cells were washed with phosphate-buffer saline at 37°C before being incubated with salicylamide (1.5 mM) to block phase II-conjugation enzymes. 7-Ethoxyresorufine or 7-Methoxyresorufine was added 1 min later and the oxidation of the substrates was measured by fluorescence detection every 2 min during 20 min at 37°C. The reaction rate was linear with time. After the assessment of P450 1A1/2 and 1A2 activities, the colorimetric WST-1 based assay was performed to normalize said activities with the quantity of viable cells. Values (pmol/min/OD) are correspond to the mean values ± standard deviation of triplicate measurements.

### Mitotic Index

To measure the mitotic index of PHH cultivated in 3D cultures established according to the method of the invention, a microtubule-depolymerizing drug, colcemid, was used to arrest cells in metaphase. Cells were treated during 24h with colcemid before being fixed in formol 4%. The quantification of the mitotic index was performed by immunostaining of the phosphorylated histone H3.

### Imaging

Observation and imaging of the cultured hepatocytes were carried out using two-photon excited fluorescence (TPEF) microscopy (also referred to as non-linear, multiphoton, or two-photon laser scanning microscopy). TPEF microscopy is an alternative to confocal and deconvolution microscopy that is particularly suited for deep and high-resolution three-dimensional imaging. TPEF enables observation of endogenous auto-fluorescent unstained or stained samples. In tandem with Second Harmonic Generation (SHG) which allows observation of hyper polarizable fibrillar proteins such as type 1 or type 3 collagen, these methods provide images of spatially resolved 3-dimensionnal structures of cells and collagen matrix. The SHG imaging system consisted of a confocal SP5 scanning head (Leica Microsystems, Mannheim, Germany) mounted on a DMI 6000 inverted microscope (Leica Microsystems) and equipped with a MAITAI femtosecond laser (Spectra Physics, Santa Clara, CA). A high NA water immersion objective (LUMFL 60 W × 1.1 NA; Olympus, Tokyo, Japan) was used. The SHG signal was collected in the forward direction using a water immersion condenser (S1, NA =0.9; Leica Microsystems). A 405-20 bandpass filter was placed before the photomultiplier tube and oil immersion objective 10x/0.4 HC PL APO oil or 20x/0.7 HC PL APO oil.

### Statistical analysis

Results are expressed as mean values ± standard deviation. Data were analyzed with two-tailed Student's t-test. Differences were considered significant when p<0.05 (*), p<0.01 (**), or p<0.001 (***). All experiments were performed at least three times.

### Results

### 3D culture in collagen matrix after pre-culture in LAP allows long term survival of adult primary human hepatocytes

As described hereinabove, primary human hepatocytes were first cultured in a low attachment plate for 15h before being embedded in collagen gels in the presence of an appropriate growth factors/cytokines cocktail. As shown on **Figures 2A-B****,** two days after inclusion in collagen gels (d2), primary human hepatocytes appeared as isolated cells or as small clusters of round cells without any membrane extension and without any particular organization (see **Figure 2B** for 3D reconstruction). These clusters had very heterogeneous sizes and were not well organized. However, as shown on **Figures 2C-D****,** seven days after inclusion in collagen gels (d7), the number of clusters of primary human hepatocytes was higher and said clusters were bigger, thus showing that the collagen matrix could influence cluster formation over culture time. As illustrated on **Figures 2E-F****,** TPEF imaging and Z stack reconstruction performed at day 7 showed that the clusters of primary human hepatocytes had organized into acini-like structures of different sizes with a hollow lumen. These acini-like structures, also referred to as hepatospheres or spheroids, were characterized by the presence of one layer of well-organized hepatocytes embedded in the collagen matrix. Fifteen days after inclusion in collagen gels (d15), SHG microscopy showed that, at a distance from the spheroids, collagen fibers appeared relaxed, with smooth randomly oriented nanofibers **(****Figure 2G****).** These collagen fibers appeared more concentrated in the close microenvironment of the spheroids and also stretched, thereby constraining the spheroid volume (see collagen signature CS-1 on **Figure 2H****).** Remarkably, parallel bundles of collagen fibers were distributed perpendicular to close spheroids (see collagen signature CS-2 on **Figure 2H****).** All these observations thus demonstrate that adult primary human hepatocytes can form acini-like structures, *i.e.,* spheroids, when embedded in a collagen gel. This process appears to be dynamic and spheroids seem able to remodel the collagen matrix over culture time.

Then, long-term survival of the primary human hepatocytes (PHH) was evaluated by analyzing a panel of pro-apoptotic and pro-survival factors. As shown on **Figure 3****,** the expression over culture time of pro-apoptotic factors (phospho-BAD, BAX, BAK, BID, BIM, PUMA) **(****Figure 3A****)** and pro-survival factors (MCL1, BCL2-XL, BCL2) **(****Figure 3B****)** was assessed in PHH either in control 2D culture or in 3D culture established as described hereinabove. The expression of most pro-apoptotic proteins assessed appeared similar in PHH in control 2D culture and in PHH in 3D culture. Only P-BAD was less phosphorylated in PHH in 3D culture than in PHH in 2D culture. By contrast, the expression of pro-survival proteins decreased rapidly in PHH in 2D cultures whereas their expression was maintained, and for some actually up-regulated (BCL2-XL, BCL2), in PHH in 3D culture. The difference in pro-survival protein expressions began to be clearly seen at day 5 and increased thereafter until at least day 15 of culture. Moreover, no cleaved-caspase 3 could be detected in PHH in 3D culture but caspase-dependent apoptosis could be induced in PHH in 3D culture by cisplatin treatment (data not shown). Taken all together, these data demonstrate that PHH in 3D culture do not undergo apoptosis.

### 3D culture in collagen matrix after pre-culture in LAP allows stable differentiation of adult primary human hepatocytes

As shown on **Figure 4****,** analysis by RT-qPCR and western blotting showed that the long-term survival of the PHH in 3D culture was associated with an increase in the expression of epithelial markers (E-cadherin) and a decrease in the expression of mesenchymal markers (N-cadherin, vimentin, cytokeratin 8, cytokeratin 18). The expression of N-cadherin and vimentin was significantly lower in PHH in 3D cultures than in PHH in control 2D cultures, stimulated by the same growth factors/cytokines, both at the mRNA level **(****Figure 4A****)** and at the protein level **(****Figure 4B****).** Notably, the expression of N-cadherin increased rapidly in PHH in control 2D culture, from day 2 of culture and thereafter. E-cadherin at the mRNA level was highly expressed in PHH in 3D culture and this expression further increased over culture time. Moreover, immuno-detection in PHH spheroids showed a distinct localization of N- and E-cadherin at the apical, lateral and basal membranes. More precisely, increased E-cadherin labeling could be clearly seen between day 5 and 15 at the apical and lateral membranes, whereas N-cadherin was located preferentially at the lateral and basal membranes in agreement with their specific localizations in hepatocytes in a human liver (data not shown). Interestingly, MKL1/MRTF-A transcription factor (megakaryoblastic leukemia 1/myocardin-related transcription factor), which has been described to be force-mediated dependent (Willer *et al.*, 2017; Flouriot *et al.* 2014), showed a nuclear localization only in PHH in 3D cultures. The transcription factor could not be detected in PHH in 2D cultures, neither in the nucleus nor in the cytoplasm (data not shown).

Taken all together, these data indicate that the protein expression in PHH in 3D culture established as described hereinabove is significantly different from that in PHH in control 2D culture and closely resembles that of *in situ* hepatocytes.

### Adult primary human hepatocytes in 3D culture in collagen matrix after a pre-culture in LAP display hepatic functions

To ascertain the impact of 3D culture on human hepatocyte differentiation, analyses by RT-qPCR were carried out to investigate the mRNA expression levels of alpha-fetoprotein, albumin, aldolase B, and phase I-II-III xenobiotic metabolism enzymes (also referred to as detoxifying enzymes). As shown on Figure 5A, the mRNA expression of alpha-fetoprotein, albumin and aldolase B was significantly higher in PHH in 3D cultures than in PHH in control 2D culture, stimulated by the same growth factors/cytokines cocktail. As shown on **Figures 5B-D****,** phase I (CYP1A2, CYP3A4, CYP2E1), phase II (GSTA1/2, UGT1A1, NAT2) and phase III (MRP2, OCT1) detoxifying enzymes were also highly expressed in PHH in 3D culture.

RT-qPCR analysis also showed a strong induction of the expression of the transcription factors involved in the regulation of detoxifying pathways (CAR, PXR and AhR) in the PHH in 3D culture as compared to that in PHH in control 2D cultures **(****Figure 5E****).** CYP1A2 (MROD) activity was significantly increased in PHH in 3D culture and, interestingly, a potentialization of 3-methylcholanthrene (3-MC) induction of CYP1A (EROD) activity was only observed in PHH in 3D culture **(****Figure 5H****).** Said potentialization demonstrates the high detoxification capacities of primary human hepatocytes embedded in collagen matrix according to the method described hereinabove. The marked 3-MC induction of CYP1A activities in PHH in 3D culture was confirmed with the observation of a significantly increased CYP1A2 mRNA expression in PHH in 3D culture as compared to that in PHH in control 2D culture, stimulated by the same growth factors/cytokines cocktail **(****Figure 5F****).** The mRNA expression of the nuclear receptor HNF4 alpha was also higher in PHH in 3D culture than that in PHH in 2D culture at days 4, 15 and 28 **(****Figure 5G****).**

As shown on **Figure 6****,** drug-transporter MRP2 localized exclusively at the apico-lateral region and the apical/bile canalicular domain. Moreover, evaluation of MRP2 functional activity using the fluorescein diacetate CDFDA assay showed a clear efflux in the central lumen of the PHH spheroids at days 4, 10, 28. Drug-transporter MRP3 localized on both apical and lateral domains.

### Formation of PHH spheroids in 3D culture in collagen matrix after a pre-culture in LAP

The size and morphology of the hepato-spheroids were assessed over two weeks of culture (Figure 7). The cells were observed after hematoxylin, eosin and saffron (HES) staining and the mean diameter of the spheroids quantified. Over the two weeks of culture, all the spheroids showed only one layer of cells and displayed an acinus-like structure, with a hollow lumen (see **Figures 6** and **7A****).**

As indicated above, the PHH forming the spheroids were polarized, as could be observed notably with the exclusive localization of the drug-transporter MRP2 at the apico-lateral region and the apical/bile canalicular domain. Moreover, as indicated above, the PHH forming the spheroids retained their differentiated state and their hepatic functions.

Expression of hypoxia and/or apoptosis markers was not detected in the spheroids obtained with the method of the invention. Moreover, the size of the spheroids did not decrease over time during the 3D culture. On the contrary, as shown on **Figures 7A-B****,** the size of the spheroids gradually increased over time.

### Proliferation of adult primary human hepatocytes in 3D culture in collagen matrix after a pre-culture in LAP

As mentioned above, the size of the spheroids gradually increased over time. Notably, the number of hepatocyte spheroids with a diameter inferior to 60 µm decreased whereas the number of spheroids with a diameter superior to 60 µm progressively increased **(****Figure 7C****).** As shown in **Table 4** below, the size quantifications indicated an increase of the mean diameter of spheroids from 47.62 µm ± 1.61 µm at day 2 to 72.04 µm ± 6.92 µm at day 10 corresponding to a 3-fold increase of the spheroid volume, approximatively. At day 15, the spheroid size stabilized or decreased slightly, staying constant thereafter **(Table 4** and results not shown).

**Table 4: quantification of the size of PHH spheroids in 3D cultures**

| Time from the start of the 3D culture | Mean diameter of the spheroids (µm) ± standard deviation |
|---|---|
| Day 2 | 47.62 ± 1.61 |
| Day 3 | 51.24 ± 3.30 |
| Day 4 | 53.45 ± 8.65 |
| Day 5 | 58.36 ± 3.80 |
| Day 6 | 64.83 ± 1.84 |
| Day 7 | 68.83 ± 4.90 |
| Day 8 | 66.72 ± 3.52 |
| Day 9 | 71.31 ± 6.12 |
| Day 10 | 72.04 ± 6.92 |
| Day 15 | 65.47 ± 7.33 |

Next, the number of Ki67 and BrdU positive cells was quantified over the first 8 days of culture. Both Ki67 and BrdU are makers of cell proliferation, and Ki67 and BrdU positive cells are cells undergoing S phase. Hepatocytes were isolated from 8 different patients (see **Table 1)** and Ki67 staining or BrdU labeling were analyzed both in 3D and 2D cultures. Albumin, a marker of mature hepatocyte function, was detected as a positive control to quantify only Alb⁺ hepatocytes. As shown on **Figures 8A** **and** **9A-B****,** the high numbers of Ki67⁺/Alb⁺ and/or BrdU⁺/Alb⁺ hepatocytes observed between days 2 and 7 demonstrated the presence of proliferative primary human hepatocytes in the 3D cultures established as described hereinabove. No Ki67⁺ hepatocytes could be detected in the control 2D cultures, stimulated by the same growth factors/cytokines cocktail. RT-qPCR analysis of the expressions of the cell cycle markers Cdk2, cyclin D1, PCNA, P21 and P27 showed that said cell cycle markers were highest expressed in 3D cultures at day 4 as compared to 3D day 2 and to 2D days 2 and 4 **(****Figure 8B****).** In summary, the analysis of 3D cultures of primary human hepatocytes from 8 different patients gave highly similar results **(****Figure 9C****),** demonstrating the presence of a high number of Ki67⁺/Alb⁺ and BrdU⁺/Alb⁺ hepatocytes with only slight variations in the kinetics of the marker expression. By contrast, the Alb⁺ hepatocytes in control 2D cultures were always Ki67⁻and BrdU⁻ (data not shown). A mean of the cumulative/additive index of Ki67⁺/Alb⁺ hepatocytes from all the experiments reached 280% (+/- 60%) indicating that primary human hepatocytes could undergo at least two cell cycles during the first week of the 3D culture established as described hereinabove.

The results clearly demonstrate for the first time that 3D cultures in collagen gels established as described hereinabove provide a microenvironment enabling adult primary human hepatocytes to proliferate, in particular in the presence of a growth factor cocktail. By contrast, PHH in control 2D cultures did not proliferate, even in the presence of the same growth factor cocktail.

Analyses of Ki67 **(****Figure 10A****)** and cyclin **D1 (****Figure 10B****)** expressions and of BrdU incorporation **(****Figure 10C****)** were performed during the first fifteen days after seeding in 3D cultures of primary human hepatocytes established as described hereinabove from seven different donors. As shown in **Figure 11A****,** primary human hepatocytes in said 3D cultures could undergo a second wave of proliferation during the second week of culture, between day 8 and 15 depending on the donor. These two successive waves of proliferation were also detected by a phospho-histone H3 immunostaining **(****Figure 11B****)** allowing the quantification of the proportion of cells blocked in the M phase of the cell cycle after 24h of treatment with colcemid. Cumulative mitotic index **(****Figure 11C****)** showed that about 300% of cells are in M phase during the first week of culture and about 200% during the second one. Thereafter, no more Ki67, cyclin D1 and BrdU positive cells could be detected (results not shown).

### Transient MEK/ERK pathway inhibition induces a new wave of proliferation

As described hereinabove, analysis of all the 3D cultures established according to the method of the invention from hepatocytes isolated from different donors (HL-a to HL-j, see **Table 1)** demonstrated the proliferation capability of adult human hepatocytes between day 2 and 15, with small variations in the proliferation response depending on the donor. No further proliferation could be detected after two weeks of culture. It was previously shown that transient activation of the MEK/ERK pathway stimulates DNA replication in rodent hepatocytes, and that inhibition of the MEK/ERK pathway blocks rodent hepatocyte proliferation. It was also shown that sustained activation of the MEK/ERK pathway can have a negative role on rat hepatocyte proliferation (Fremin *et al.*, 2009) and that overactivation of the cascade can also inhibit cell replication in the human hepatocarcinoma cell line HuH-7 (Guegan *et al.*, 2015). Proliferation of the primary human hepatocytes was assessed after the MAPK MEK 1/2-ERK 1/2 pathway was transiently inhibited for 48 h with the MEK inhibitor U0126 in 3D cultures established as described hereinabove at day 12 or 15. Control of ERK1/2 phosphorylation confirmed a strong inhibition of ERK1/2 activity at the end of the U0126 treatment in all culture conditions **(****Figure 12A****).** Following removal of the MEK inhibitor, cumulative Ki67 **(****Figure 12B****)** and cyclin D1 **(****Figure 12C****)** positive cells indicated that 50 % to 70 % of the cells could undergo a new cell cycle (Ki67⁺/Alb⁺ and cyclinD1⁺/Alb⁺). No positive cells could be detected in DMSO control experiments or in the control 2D cultures after a similar transient MEK/ERK inhibition (data not shown).

### Example 2:

### Materials and Methods

### Material

### Cells

Primary human hepatocytes (PHH) were obtained as described hereinabove.

### Methods

### Primary human hepatocyte culture

Primary human hepatocytes 3D cultures were set up as described hereinabove (see Example 1). Briefly, PHH were first cultured in a low attachment plate. Then, the aggregates of PHH thus obtained were embedded in a collagen matrix and further cultured in the collagen matrix.

### Culture medium

The PHH were cultured in WE HH medium, defined hereinabove (see Example 1).

### Immunohistochemistry

Immuno-histochemical staining was performed as described hereinabove to assess the proliferation of the PHH.

### Results

The importance of the growth factor stimulation for the proliferation of PHH in the 3D culture according to the invention was further studied. PHH in collagen matrix were cultured as described hereinabove in WE HH medium depleted of EGF, HGF, and/or ITS (insulin, transferrin and sodium selenite). PHH in collagen matrix were also cultured as described hereinabove in WE HH medium depleted of FCS. As shown in **Table 5** below, the proliferation of PHH cultured in the different culture media was assessed by quantification of the number of Cyclin D1⁺/Alb⁺ cells with respect to the percentage of positive cells in the culture control (WE HH medium) and related to the number of cells detected.

**Table 5: Percentage of proliferating PHH in different culture media with respect to the control culture condition (WE HH medium)**

| Culture conditions | % of proliferation |
|---|---|
| Control (WE HH medium) | 100 |
| Hydro N | 99.9 |
| -EGF | 73.2 |
| -HGF | 86.8 |
| -ITS | 70.2 |
| -EGF/-HGF/-ITS | 66.4 |
| -FCS | 58 |

| | |
|---|---|
| Data are from one experiment representative of three. | |

When grown in WE HH medium comprising hydrocortisone at a concentration of 50 µM ("Hydro N"), *i.e.,* a concentration 50-fold higher than that in the control condition, PPH displayed a percentage of proliferation similar to that observed when they were grown in the control condition. Single depletion of each of the growth factor present in the cocktail ("-EGF", "-HGF" or "-ITS") only resulted in a partial decrease in PHH proliferation (at most a 30% decrease). Moreover, neither the concomitant depletion of EGF, HGF and ITS ("-EGF/-HGF/-ITS") nor the single depletion of fetal calf serum ("-FCS") abolished the proliferation of PHH. These results thus demonstrate that the PHH can proliferate in 3D cultures according to the invention even in absence of EGF, in the absence of HGF, and/or in the absence of ITS. Strikingly, the observed results also demonstrate that the PHH can proliferate in 3D cultures according to the invention even in absence of fetal calf serum (FCS).

The effect of collagen stiffness on the proliferation of PHH was also assessed. As shown in **Table 6** below, the proliferation of PHH cultured in the indicated collagen matrices was assessed by quantification of the number of Cyclin D1⁺/Alb⁺ cells with respect to the percentage of positive cells in the collagen matrix control (1.5 mg/mL) and related to the number of cells detected. Increasing type I collagen concentration from 1.5 mg/mL to 3 mg/mL or 4 mg/mL induced a decrease in PHH proliferation of about 50% and 80%, respectively. By contrast, decreasing type I collagen concentration from 1.5 mg/mL to 0.75 mg/mL did not induce a decrease in PHH proliferation. These results thus demonstrate that increasing the collagen concentration of the 3D matrix, and thus the matrix stiffness, inhibits the proliferation of hepatocytes in a concentration-dependent manner.

**Table 6: Percentage of proliferating PHH in different culture conditions with respect to the control culture condition (1.5 mg/mL collagen)**

| Culture conditions | % of proliferation |
|---|---|
| 1.5 mg/mL collagen | 100 |
| 0.75mg/mL collagen | 100 |
| 3 mg/mL collagen | 49.9 |
| 4 mg/mL collagen | 17.6 |
| No incubation in a LAP | 16.9 |
| Incubation in a LAP not followed by 3D culture in collagen matrix | 11.7 |

Strikingly, PHH embedded directly in 3D collagen gels and cultured in WE HH medium without a prior incubation in a low attachment plate displayed a very low rate of proliferation (16.9%, see **Table 6).** Similarly, PHH cultured in a low attachment plate in WE HH medium without ever being embedded in a 3D collagen matrix displayed a very low rate of proliferation (11.7%, see **Table 6).**

Taken all together, these results demonstrate that the 3D microenvironment and controlled collagen stiffness are major factors for inducing human adult primary hepatocyte proliferation. Moreover, these results show that both a first incubation (or culture) of the PHH in a non-adherent culture vessel, *e.g.*, a low attachment plate, and the subsequent inclusion of the PHH in a collagen matrix are required to induce the proliferation of adult PHH.

### Example 3:

### Materials and Methods

### Material

### Cells

Primary human hepatocytes (PHH) were obtained as described hereinabove (see Example 1).

### Methods

### Primary human hepatocyte culture

Primary human hepatocytes 2D and 3D cultures in a collagen matrix were set up as described hereinabove (see Example 1). PHH were cultured in WE HH medium as described hereinabove.

### Comet Assay

PHH either in control 2D culture (2D) or in 3D culture established according to the method of the invention in a collagen matrix (3D) were incubated with the indicated genotoxic drugs at the indicated concentrations (see **Table 7)** from the start of the culture. After 9 days, PHH were extracted from the collagen matrix by the action of purified collagenase. The cell pellets were resuspended in 0.5% low-melting point agarose and laid on conventional microscope slides covered with regular agarose. The electrophoresis migration was processed and after staining with propidium iodide, at least 100 images were acquired using a fluorescence microscope. The images were analyzed using the Comet Assay IV software. The extent of DNA damage in individual cells was evaluated by the percentage of tail DNA.

**Table 7: genotoxic drugs**

| | Concentration 1 | Concentration 2 |
|---|---|---|
| Cisplatin (CIS) | 5 µg/mL | 10 µg/mL |
| AFB1 | 0.1 µM | 0.5µM |
| 4-ABP | 1 µM | 10 µM |

### γH2Ax immunostaining after treatment of PHH

PHH in 3D culture in a collagen matrix established according to the method of the invention (3D) were incubated with the indicated genotoxic drugs at the indicated concentrations (see **Table 7)** from the start of the culture. After 9 days, immuno-histochemical staining was performed as described hereinabove to detect the presence of phosphorylated H2AX (*i.e.*, γH2Ax), a marker of DNA double-strand break, in the PHH.

### Results

To assess the sensitivity of the spheroids comprising proliferative PHH as a model for testing drug-induced genotoxicity, the effects of two classes of compounds on said spheroids were studied: alkylating agents, *i.e.,* cisplatin (phosphorylated histone γH2Ax assay, see **Figure 14****),** that exert direct effect on DNA without being metabolized; 2 proven carcinogens, *i.e.,* 4-ABP (4-aminobiphenyl) and AFB-1 (aflatoxin B1) that become genotoxic only after metabolic activation **(****Figures 13** and **14****).** The primary human hepatocytes were incubated for 9 days with the genotoxic drugs from the start of the 3D culture, during a period when they proliferate.

To compare the induced genotoxicity of these compounds in control 2D culture (2D) or in 3D culture established according to the method of the invention in a collagen matrix (3D), comet assay was performed. In 2D cultures, 4-ABP and AFB1 caused only very low levels of damages (about 3 %). By contrast, in 3D culture established according to the method of the invention in a collagen matrix, AFB-1 caused high and dose-dependent levels of DNA damages (up to about 25 %) and 4-ABP 1 caused higher (about the double) and dose-dependent levels of DNA damages **(****Figure 13****).**

To confirm these results, the number of γH2Ax positive cells in 3D cultures established according to the method of the invention in a collagen matrix was quantified by immunostaining. Very high levels of DNA damages were thus observed with the 3 tested drugs: cisplatin induced DNA damages in about 90 % of the PHH, 4-ABP in about 50 % of the PHH, and AFB1 in about 35% of the PHH **(****Figure 14****).**

These results thus demonstrate that, with their highly differentiated and proliferating status, the spheroid comprising proliferative PHH constitute a relevant model for testing the genotoxicity of environmental and chemical compounds.

### Example 4:

### Materials and Methods

### Material

### Cells

Primary human hepatocytes (PHH) were obtained as described hereinabove (see Example 1).

### Reagents

Methacrylated gelatin was obtained from the ART Bio-encres (Accélérateur de Recherches Technologiques de l'Inserm, Bordeaux). Lithium phenyl-2,4,6-trimethylbenzoylphosphinate (LAP) was obtained from TCI.

### Methods

### Primary human hepatocyte culture in gelatin methacrylate (GelMa)

In a manner similar to that shown on **Figure 1****,** primary human hepatocytes (PHH) isolated as indicated above (see Example 1) were first cultured in a low attachment plate **(****Figure 1A****).** Then, the aggregates of PHH thus obtained were embedded in a GelMa matrix (also referred to as GelMa hydrogel) and further cultured in the GelMa matrix **(****Figure 1B****).**

*Culture in a low attachment plate*: as indicated above (see Example 1), isolated human hepatocytes were incubated at 37°C, 5% CO2, humidity 100% during two days (about 48h) in 6-well low attachment plates (Corning^{®}, Costar^{®}) at a concentration of about 3x10⁶ cells well, in a modified William's E medium comprising gentamicin (50 µg/mL), penicillin (100 U/mL), streptomycin (100 µg/mL), insulin (5 µg/mL), L-glutamine (2 mM), albumin (0.1% (w/v)) and with or without FCS (fetal calf serum) (10% (v/v)). *Preparation of medium comprising 5% GelMa:* small crushed fragments of freeze-dried GelMa (up to 2 mm in length) were dissolved at 37°C for a minimum of 8h in a modified William's E medium comprising gentamicin (50 µg/mL), penicillin (100 U/mL), streptomycin (100 mg/mL), insulin (15 µg/mL), L-glutamine (2 mM), albumin (0.1% (w/v)), transferrin (5.5 µg/mL), sodium selenite (5 µg/mL), hydrocortisone (1 µM), HGF (hepatocyte growth factor) (2.5 ng/mL), EGF (epidermal growth factor) (0.05 ng/µL) and with or without FCS (fetal calf serum) (10% (v/v)). A volume of lithium phenyl-2,4,6-trimethylbenzoylphosphinate (LAP) at a concentration of 10 mg/mL was added to obtain a modified William's E medium comprising concentration of GelMa of 5 g/100 mL, *i.e.*, a final concentration of 5% GelMa and a final concentration of lithium phenyl-2,4,6-trimethylbenzoylphosphinate of 100 mg/mL, *i.e.,* a final concentration of 0.1% LAP. Before use, the medium comprising 5% GelMa and 0.1% LAP was kept at 37°C, protected from light.

*Inclusion in a GelMA matrix followed by culture of hepatocytes embedded in the collagen matrix*: human hepatocytes were transferred to a suitable container such as an Eppendorf tube (1.5 or 2 mL) or a Falcon tube (15 mL) and centrifuged at 200 g for 2 min. The supernatant was removed and medium comprising 5% GelMa and 0.1% LAP was added to obtain a concentration of about 10⁶ human hepatocytes per mL of GelMa matrix, *i.e.*, of medium comprising 5% GelMa and 0.1% LAP. 100 µL of medium comprising 5% GelMa, 0.1% LAP and human hepatocytes were added to the wells of a 96-well plate. Alternatively, 300 µL of medium comprising 5% GelMa, 0.1% LAP and human hepatocytes were added to the wells of a 48-well plate. Polymerization of the GelMa matrix was induced by illumination for 30 seconds to 10 min with a 365, 405 nm or 530 nm LED, preferably by illumination for 60 seconds with a 405 nm LED. After polymerization of the GelMa matrix, a volume of the same modified William's E medium equal to the volume of GelMa matrix was added. The hepatocytes embedded in the GelMa matrix were cultured in the modified William's E medium as described above for at least 2 days at 37°C, 5% CO2, humidity 100%. The medium was changed every 48h.

### Results

### Formation of PHH spheroids in 3D culture in GelMa matrix after a pre-culture in LAP

The size and morphology of the hepato-spheroids was assessed over two weeks of culture **(****Figure** 15). The cells were observed after hematoxylin, eosin and saffron (HES) staining. Over the two weeks of culture, all the spheroids showed only one layer of cells and displayed an acinus-like structure, with a hollow lumen (see **Figures 15** and **16****).**

As with the PHH spheroids cultured in a collagen matrix, the PHH forming the spheroids cultured in a GelMa matrix were polarized, as could be observed notably with the exclusive localization of the drug-transporter MRP2 at the apico-lateral region and the apical/bile canalicular domain.

### Proliferation of adult primary human hepatocytes in GelMa matrix after a pre-culture in LAP

Proliferation of the primary human hepatocytes in GelMa matrix was assessed through the analyses of cyclin D1 and albumin (Alb) expression over 30 days after seeding in 3D cultures established according to the method of the invention in a GelMa matrix **(****Figure 17****).**

Alb⁺/cyclin D1⁺ PHH could be detected from day 2, demonstrating that PHH were able to proliferate in 3D cultures established according to the method of the invention in a GelMa matrix. As shown on **Figure 17****,** during the wave of proliferation, a maximum level of proliferation of about 60% was observed.

## Claims

1. A method of culturing primary human hepatocytes (PHH) to obtain spheroids comprising proliferative PHH, said method comprising:
a) first culturing the PHH in a non-adherent culture vessel;
b) then transferring the PHH to a culture medium comprising collagen at a concentration ranging from about 0.75 mg/mL to about 3 mg/mL or gelatin at a concentration ranging from about 15% (w/v) to about 40% (w/v) or methacrylated gelatin (GelMa) at a concentration ranging from about 1% (w/v) to about 10% (w/v), thereby embedding the PHH in a collagen matrix or in a gelatin matrix; and
c) culturing the PHH embedded in the collagen matrix or in the gelatin matrix;
thereby obtaining spheroids comprising proliferative PHH, wherein said spheroids have an acinus-like structure with a hollow lumen.

2. The method according to claim **1,** wherein at step b) the PHH are transferred to a culture medium comprising fibrillar collagen selected from the group consisting of type I collagen, type II collagen, type III collagen, type V collagen, type VI collagen, type XI collagen, type XXIV collagen, type XXVII collagen and any mixtures thereof.

3. The method according to claim **1** or **2,** wherein at step b) the PHH are transferred to a culture medium comprising fibrillar collagen selected from the group consisting of type I collagen, type III collagen, and any mixtures thereof.

4. The method according to claim **1,** wherein at step b) the PHH are transferred to a culture medium comprising methacrylated gelatin (GelMa) at a concentration of about 5% (w/v).

5. The method according to any one of claims **1** to **4,** wherein at step a) the PHH are cultured in a non-adherent culture vessel for a period ranging from about 1h to about 96h.

6. The method according to any one of claims **1** to **5,** wherein at step a) the non-adherent culture vessel is a low or ultra-low attachment culture vessel **characterized by** the presence of a coating that prevents cell attachment to the inner surface of the culture vessel.

7. The method according to any one of claims **1** to **6,** wherein at step c) the PHH embedded in the collagen or gelatin matrix are cultured for at least about 2 days.

8. The method according to any one of claims **1** to **7,** further comprising:
d) adding an inhibitor of the MAPK MEK1/2-ERK1/2 pathway to the culture of PHH embedded in the collagen or gelatin matrix, thereby inducing one or more additional wave(s) of proliferation of the PHH.

9. The method according to any one of claims **1** to **8,** further comprising:
e) incubating the spheroids comprising proliferative PHH embedded in the collagen or gelatin matrix with a collagenase or an enzyme mixture with collagenolytic activity, thereby isolating the spheroids comprising proliferative PHH from the collagen or gelatin matrix.

10. A spheroid comprising proliferative primary human hepatocytes (PHH) embedded in a collagen or gelatin matrix susceptible to be obtained with the method according to any one of claims **1** to **8,** wherein said spheroid has an acinus-like structure with a hollow lumen.

11. The spheroid according to claim **10,** wherein said spheroid has a diameter ranging from about 20 µm to about 130 µm.

12. The spheroid according to claim **10** or **11,** wherein said spheroid comprises from about 5 to about 100 proliferative PHH.

13. Use of the spheroid according to any one of claims **10** to **12** for engineering an artificial liver model or an artificial liver organ.

14. Use of the spheroid according to any one of claims **10** to **12** for assessing *in vitro* the liver toxicity, in particular liver genotoxicity, and/or the effects of a drug or a compound.

15. An *in vitro* method of assessing the toxicity and/or the effects of a drug or a compound, the method comprising:
a) obtaining spheroids comprising proliferative primary human hepatocytes (PHH) according to the method of any one of claims **1** to **9,** wherein said spheroids have an acinus-like structure with a hollow lumen;
b) contacting the spheroids comprising proliferative PHH with a drug or a compound; and
c) assessing the toxicity, genotoxicity and/or the effects of the drug or the compound on the spheroids comprising proliferative PHH.

## Patentansprüche

1. Verfahren zum Kultivieren primärer humaner Hepatozyten (PHH) zur Gewinnung von Sphäroiden, die proliferatives PHH umfassen, wobei das Verfahren Folgendes umfasst:
a) erstmaliges Kultivieren der PHH in einem nicht adhärenten Kulturgefäß;
b) anschließendes Übertragen der PHH in ein Kulturmedium, das Kollagen in einer Konzentration von etwa 0,75 mg/ml bis etwa 3 mg/ml oder Gelatine in einer Konzentration von etwa 15 % (w/v) bis etwa 40 % (w/v) oder methacrylierte Gelatine (GelMa) in einer Konzentration von etwa 1 % (w/v) bis etwa 10 % (w/v) umfasst, wodurch die PHH in eine Kollagenmatrix oder in eine Gelatinematrix eingebettet werden; und
c) Kultivieren der in der Kollagenmatrix oder in der Gelatinematrix eingebetteten PHH;
dadurch Erhalten von Sphäroiden, die proliferative PHH umfassen, wobei die Sphäroide eine azinusartige Struktur mit einem Hohllumen aufweisen.

2. Verfahren nach Anspruch **1,** wobei in Schritt b) die PHH in ein Kulturmedium übertragen werden, das fibrilläres Kollagen umfasst, ausgewählt aus der Gruppe bestehend aus Kollagen Typ I, Kollagen Typ II, Kollagen Typ III, Kollagen Typ V, Kollagen Typ VI, Kollagen Typ XI, Kollagen Typ XXIV, Kollagen des Typs XXVII und deren Gemische.

3. Verfahren nach Anspruch **1** oder **2,** wobei in Schritt b) die PHH in ein Kulturmedium übertragen werden, das fibrilläres Kollagen umfasst, ausgewählt aus der Gruppe bestehend aus Kollagen Typ I, Kollagen Typ III und deren Gemische.

4. Verfahren nach Anspruch **1,** wobei in Schritt b) die PHH in ein Kulturmedium übertragen werden, das methacrylierte Gelatine (GelMa) in einer Konzentration von etwa 5 % (w/v) umfasst.

5. Verfahren nach einem der Ansprüche **1** bis **4,** wobei in Schritt a) die PHH in einem nicht adhärenten Kulturgefäß für einen Zeitraum von etwa 1 h bis etwa 96 h kultiviert werden.

6. Verfahren nach einem der Ansprüche **1** bis **5,** wobei in Schritt a) das nicht adhärente Kulturgefäß ein Kulturgefäß mit niedriger oder ultraniedriger Haftung ist, das durch das Vorhandensein einer Beschichtung gekennzeichnet ist, die eine Zellhaftung an der Innenoberfläche des Kulturgefäßes verhindert.

7. Verfahren nach einem der Ansprüche **1** bis **6,** wobei in Schritt c) die in die Kollagen- oder Gelatinematrix eingebetteten PHH für mindestens etwa 2 Tage kultiviert werden.

8. Verfahren nach einem der Ansprüche 1 bis 7, ferner umfassend:
d) Hinzufügen eines Inhibitors des MAPK MEK1/2-ERK1/2-Pfads zur Kultur von in der Kollagen- oder Gelatinematrix eingebetteten PHH, wodurch eine oder mehrere zusätzliche Wellen der Proliferation der PHH induziert werden.

9. Verfahren nach einem der Ansprüche **1** bis **8,** ferner umfassend:
e) Inkubieren der in die Kollagen- oder Gelatinematrix eingebetteten proliferativen PHH umfassenden Sphäroide mit einer Kollagenase oder einer Enzymmischung mit kollagenolytischer Aktivität, wodurch die proliferativen PHH umfassenden Sphäroide von der Kollagen- oder Gelatinematrix isoliert werden.

10. Sphäroid, umfassend proliferative primäre humane Hepatozyten (PHH), eingebettet in eine Kollagen- oder Gelatinematrix, die durch das Verfahren nach einem der Ansprüche **1** bis **8** erhalten werden, wobei das Sphäroid eine azinusartige Struktur mit einem Hohllumen aufweist.

11. Sphäroid nach Anspruch **10,** wobei das Sphäroid einen Durchmesser im Bereich von etwa 20 µm bis etwa 130 µm aufweist.

12. Sphäroid nach Anspruch **10** oder **11,** wobei das Sphäroid etwa 5 bis etwa 100 proliferative PHH umfasst.

13. Verwendung des Sphäroids nach einem der Ansprüche **10** bis **12** zum Herstellen eines künstlichen Lebermodells oder eines künstlichen Leberorgans.

14. Verwendung des Sphäroids nach einem der Ansprüche **10** bis **12** zum *In-vitro-*Beurteilen der Lebertoxizität, insbesondere der Lebergentoxizität, und/oder der Wirkungen eines Arzneimittels oder einer Verbindung.

15. In-vitro-Verfahren zum Beurteilen der Toxizität und/oder der Wirkungen eines Arzneimittels oder einer Verbindung, das Verfahren umfassend:
a) Erhalten von Sphäroiden, die proliferierende primäre menschliche Hepatozyten (PHH) nach dem Verfahren nach einem der Ansprüche **1** bis **9** umfassen, wobei diese Sphäroide eine azinusartige Struktur mit einem Hohllumen aufweisen;
b) Kontaktieren der Sphäroide, die proliferierende PHH umfassen, mit einem Arzneimittel oder einer Verbindung; und
c) Beurteilen der Toxizität, Gentoxizität und/oder der Wirkungen des Arzneimittels oder der Verbindung auf die Sphäroide, die proliferative PHH umfassen.

## Revendications

1. Un procédé de culture d'hépatocytes humains primaires (HHP) pour obtenir des sphéroïdes comprenant des HHP proliférants, ledit procédé comprenant :
a) premièrement la culture des HHP dans un récipient de culture non adhérent ;
b) puis le transfert des HHP dans un milieu de culture comprenant du collagène à une concentration allant d'environ 0,75 mg/mL à environ 3 mg/mL ou de la gélatine à une concentration allant d'environ 15 % (m/v) à environ 40 % (m/v) ou de la gélatine méthacrylée (GelMa) à une concentration allant d'environ 1 % (m/v) à environ 10 % (m/v), incorporant ainsi les HHP dans une matrice de collagène ou dans une matrice de gélatine ; et
c) la culture des HHP incorporés dans la matrice de collagène ou dans la matrice de gélatine ;
obtenant ainsi des sphéroïdes comprenant des HHP proliférants, lesdits sphéroïdes ayant une structure de type acinus avec une lumière creuse.

2. Le procédé selon la revendication **1,** dans lequel, à l'étape b), les HHP sont transférés dans un milieu de culture comprenant du collagène fibrillaire sélectionné dans le groupe consistant en du collagène de type I, du collagène de type II, du collagène de type III, du collagène de type V, du collagène de type VI, du collagène de type XI, du collagène de type XXIV, du collagène de type XXVII et tout mélange de ceux-ci.

3. Le procédé selon la revendication **1** ou **2,** dans lequel, à l'étape b), les HHP sont transférés dans un milieu de culture comprenant du collagène fibrillaire sélectionné dans le groupe consistant en du collagène de type I, du collagène de type III et tout mélange de ceux-ci.

4. Le procédé selon la revendication **1,** dans lequel, à l'étape b), les HHP sont transférés dans un milieu de culture comprenant de la gélatine méthacrylée (GelMa) à une concentration d'environ 5 % (m/v).

5. Le procédé selon l'une quelconque des revendications **1** à **4,** dans lequel, à l'étape a), les HHP sont cultivés dans un récipient de culture non adhérent pendant une période allant d'environ 1 heure à environ 96 heures.

6. Le procédé selon l'une quelconque des revendications **1** à **5,** dans lequel, à l'étape a), le récipient de culture non adhérent est un récipient de culture à faible ou ultra-faible adhérence **caractérisé par** la présence d'un revêtement qui empêche l'adhérence des cellules à la surface interne du récipient de culture.

7. Le procédé selon l'une quelconque des revendications **1** à **6,** dans lequel, à l'étape c), les HHP incorporés dans la matrice de collagène ou de gélatine sont cultivés pendant au moins environ 2 jours.

8. Le procédé selon l'une quelconque des revendications 1 à 7, comprenant en outre : d) l'ajout d'un inhibiteur de la voie MAPK MEK1/2-ERK1/2 à la culture de HHP incorporés dans la matrice de collagène ou de gélatine, induisant ainsi une ou plusieurs vague(s) additionnelle(s) de prolifération des HHP.

9. Le procédé selon l'une quelconque des revendications 1 à 8, comprenant en outre : e) l'incubation des sphéroïdes comprenant des HHP proliférants incorporés dans la matrice de collagène ou de gélatine avec une collagénase ou un mélange d'enzymes ayant une activité collagénolytique, isolant ainsi les sphéroïdes comprenant des HHP proliférants de la matrice de collagène ou de gélatine.

10. Un sphéroïde comprenant des hépatocytes humains primaires (HHP) proliférants incorporés dans une matrice de collagène ou de gélatine susceptible d'être obtenu par le procédé selon l'une quelconque des revendications **1** à **8,** dans lequel ledit sphéroïde a une structure de type acinus avec une lumière creuse.

11. Le sphéroïde selon la revendication **10,** dans lequel ledit sphéroïde a un diamètre allant d'environ 20 µm à environ 130 µm.

12. Le sphéroïde la revendication **10** ou **11,** dans lequel ledit sphéroïde comprend d'environ 5 à environ 100 HHP proliférants.

13. Utilisation du sphéroïde selon l'une quelconque des revendications **10** à **12** pour la conception d'un modèle de foie artificiel ou d'un organe de foie artificiel.

14. Utilisation du sphéroïde selon l'une quelconque des revendications **10** à **12** pour l'évaluation *in vitro* de la toxicité hépatique, en particulier la génotoxicité hépatique, et/ou les effets d'un médicament ou d'un composé.

15. Un procédé *in vitro* d'évaluation de la toxicité et/ou des effets d'un médicament ou d'un composé, le procédé comprenant :
a) l'obtention de sphéroïdes comprenant des hépatocytes humains primaires (HHP) proliférants selon le procédé de l'une quelconque des revendications **1** à **9,** dans lequel lesdits sphéroïdes ont une structure de type acinus avec une lumière creuse ;
b) la mise en contact des sphéroïdes comprenant des HHP proliférants avec un médicament ou un composé ; et
c) l'évaluation de la toxicité, de la génotoxicité et/ou des effets du médicament ou du composé sur les sphéroïdes comprenant des HHP proliférants.
